# EUROPEAN PATENT APPLICATION

(11) **EP 4 227 289 A1**
(43) Date of publication of application: **16.08.2023**
(21) Application number: 21917918.1
(22) Date of filing: 17.12.2021
(51) Int. Cl.: C07C 211/61, H01L 51/00, C09D 11/06, C09D 201/00, H01L 51/50, H01L 51/56

(54) **NOVEL COMPOUND, COATING COMPOSITION COMPRISING SAME, ORGANIC LIGHT-EMITTING DEVICE USING SAME AND MANUFACTURING METHOD THEREFOR**

(30) Priority: 11.01.2021 KR 20210003017
(71) Applicant: Lg Chem, Ltd., Seoul 07336 (KR)
(72) Inventor: SEO, Minseob, Daejeon 34122 (KR); LEE, Dongwhan, Daejeon 34122 (KR); PARK, Youngju, Daejeon 34122 (KR); CHOI, Doowhan, Daejeon 34122 (KR); YOO, Seung Jun, Daejeon 34122 (KR); BAE, Jaesoon, Daejeon 34122 (KR); LEE, Jaechol, Daejeon 34122 (KR)
(74) Representative: Hoffmann Eitle
(86) International application number: PCT/KR2021/019282
(87) International publication number: WO 2022/149756

(57) **Abstract**

The present disclosure relates to a compound represented by Chemical Formula 1, a coating composition including the compound represented by Chemical Formula 1, an organic light emitting device using the same, and a method for manufacturing the same.

## Description

### [Technical Field]

The present disclosure claims priority to and the benefits of Korean Patent Application No. 10-2021-0003017, filed with the Korean Intellectual Property Office on January 11, 2021, the entire contents of which are incorporated herein by reference.

The present disclosure relates to a novel compound, a coating composition including the compound, an organic light emitting device formed using the coating composition, and a method for manufacturing the same.

### [Background Art]

An organic light emission phenomenon is one of examples converting a current to visible light by an internal process of specific organic molecules. A principle of an organic light emission phenomenon is as follows. When an organic material layer is placed between an anode and a cathode and a current is applied between the two electrodes, electrons and holes are injected to the organic material layer from the cathode and the anode, respectively. The holes and the electrons injected to the organic material layer recombine to form excitons, and light emits when these excitons fall back to the ground state. An organic electroluminescent device using such a principle may be generally formed with a cathode, an anode, and an organic material layer placed therebetween, for example, a hole injection layer, a hole transfer layer, a light emitting layer, an electron injection layer and an electron transfer layer.

A deposition process has been mainly used in the art to manufacture an organic light emitting device. However, manufacturing an organic light emitting device using a deposition process has problems in that it causes much material loss and manufacturing a large area device is difficult, and a device using a solution process has been developed in order to resolve these problems.

Accordingly, development of materials for a solution process has been required.

### Prior Art Documents

### Patent Documents

(Patent Document 1) Korean Patent Application Laid-Open Publication No. 10-2000-0051826

### [Disclosure]

### [Technical Problem]

The present disclosure is directed to providing a novel compound, a coating composition including the same, an organic light emitting device including the same, and a method for manufacturing the same.

### [Technical Solution]

One embodiment of the present disclosure provides a compound represented by the following Chemical Formula 1.

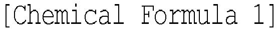

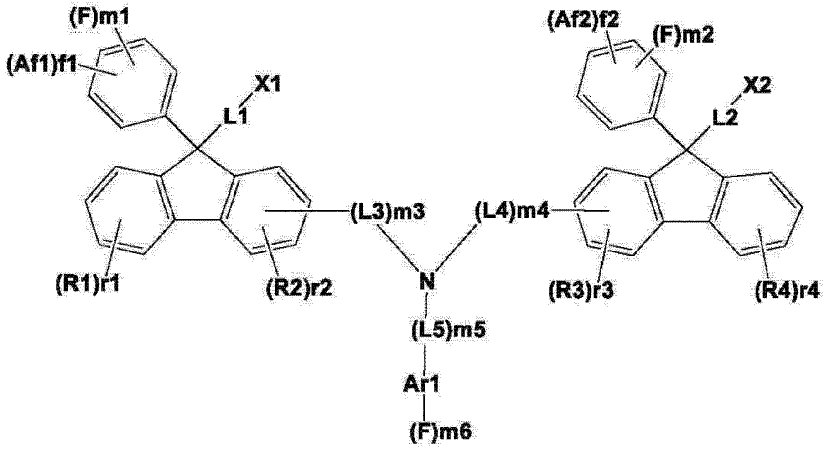

In Chemical Formula 1,
L1 and L2 are the same as or different from each other, and each independently a direct bond; a substituted or unsubstituted alkylene group; or a substituted or unsubstituted arylene group,
L3 to L5 are the same as or different from each other, and each independently a direct bond; or a substituted or unsubstituted arylene group,
R1 to R4 are the same as or different from each other, and each independently hydrogen; deuterium; a substituted or unsubstituted alkyl group; a substituted or unsubstituted alkoxy group; a substituted or unsubstituted aryl group; or a substituted or unsubstituted heteroaryl group,
Af1 and Af2 are the same as or different from each other, and each independently a substituted or unsubstituted alkyl group; or a substituted or unsubstituted aryl group,
Ar1 is a substituted or unsubstituted aryl group,
X1 and X2 are the same as or different from each other and each independently -(R101)s; or -Y-A, and at least one of X1 and X2 is -Y-A,
R101 is hydrogen; deuterium; a halogen group; a substituted or unsubstituted alkyl group; a substituted or unsubstituted alkoxy group; a substituted or unsubstituted aryl group; or a substituted or unsubstituted aryloxy group,
s is an integer of 0 to 5, and when s is 2 or greater, the two or more R101s are the same as or different from each other,
Y is a direct bond, O or S,
A is a curing group,
r1 and r4 are each an integer of 0 to 4,
r2 and r3 are each an integer of 0 to 3,
m1+m2 is an integer of 2 to 10,
m3 to m5 are each an integer of 0 to 2,
m6 is an integer of 0 to 5,
f1 and f2 are each an integer of 0 to 5, f1+m1 is 5 or less, and f2+m2 is 5 or less,
when r1 to r4 are each 2 or greater, substituents in the parentheses are the same as or different from each other, and
when m3 to m5 are each 2, substituents in the parentheses are the same as or different from each other.

Another embodiment of the present disclosure provides a coating composition including the compound.

In addition, another embodiment of the present disclosure provides an organic light emitting device including a first electrode; a second electrode; and one or more organic material layers provided between the first electrode and the second electrode, wherein one or more layers of the one or more organic material layers include the coating composition described above or a cured material thereof.

In addition, another embodiment of the present disclosure provides a method for manufacturing an organic light emitting device, the method including preparing a first electrode; forming one or more organic material layers on the first electrode; and forming a second electrode on the one or more organic material layers, wherein the forming of one or more organic material layers includes forming one or more organic material layers using the coating composition.

### [Advantageous Effects]

A compound of Chemical Formula 1 of the present disclosure can be used as a material of an organic material layer of an organic light emitting device, and, while obtaining a device having low driving voltage and excellent light emission efficiency and lifetime properties, a device having a large area can be manufactured by using a solution process.

Forming a coating composition by dissolving the compound of Chemical Formula 1 in an organic solvent according to one embodiment of the present disclosure has advantages in that the coating composition has proper viscosity for a solution process, and the compound of Chemical Formula 1 is not crystallized in the coating composition by having excellent solubility for the organic solvent.

In addition, when forming an organic material including the compound of Chemical Formula 1 of the present disclosure and then forming an organic material layer of the upper layer through a solution process, the organic material layer including the compound of Chemical Formula 1 is not dissolved in a solvent of the solution process of the upper layer, which leads to an advantage of not declining device performance.

### [Description of Drawings]

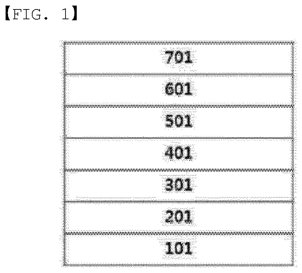

FIG. 1 illustrates an organic light emitting device according to one embodiment of the present disclosure.

### [Reference Numeral]

101: Substrate
201: First Electrode
301: Hole Injection Layer
401: Hole Transfer Layer
501: Light Emitting Layer
601: Electron Injection and Transfer Layer
701: Second Electrode

### [Mode for Disclosure]

Hereinafter, the present disclosure will be described in detail.

In the present disclosure, a description of a certain member (layer) being placed "on" another member (layer) includes not only a case of the certain member (layer) being in contact with the another member but a case of still another member (layer) being present between the two members (layers).

In the present specification, a description of a certain part "including" certain constituents means capable of further including other constituents, and does not exclude other constituents unless particularly stated on the contrary.

In the present disclosure, the "layer" has a meaning compatible with a `film' mainly used in the art, and means coating that covers a target area. The size of the "layer" is not limited, and each "layer" may have the same or a different size. According to one embodiment, the size of the "layer" may be the same as the whole device, may correspond to the size of a specific functional area, or may be as small as a single subpixel.

In the present disclosure, a "curing group" may mean a group capable of inducing crosslinking through heat treatment and/or exposure to light, or a reactive substituent crosslinking compounds and the like. The crosslinking may be produced by linking radicals generated while a carbon-carbon multiple bond or a cyclic structure is decomposed by heat treatment or light irradiation.

According to one embodiment of the present disclosure, the curing group may be selected from among the following structures.

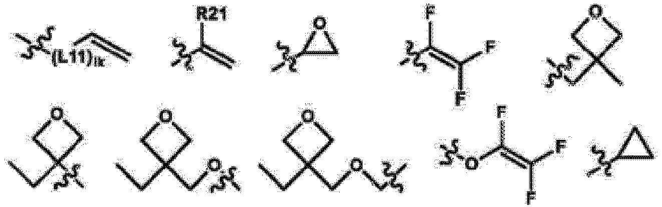

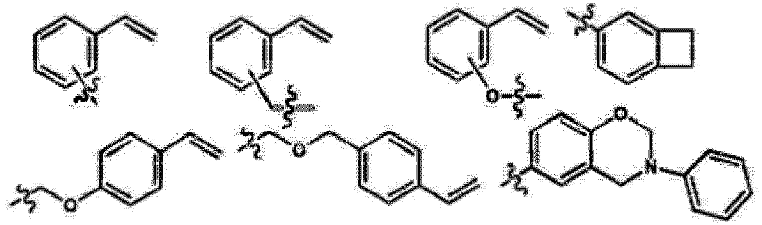

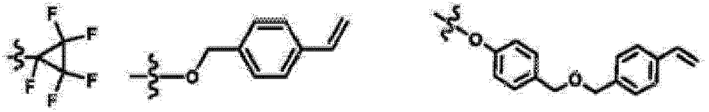

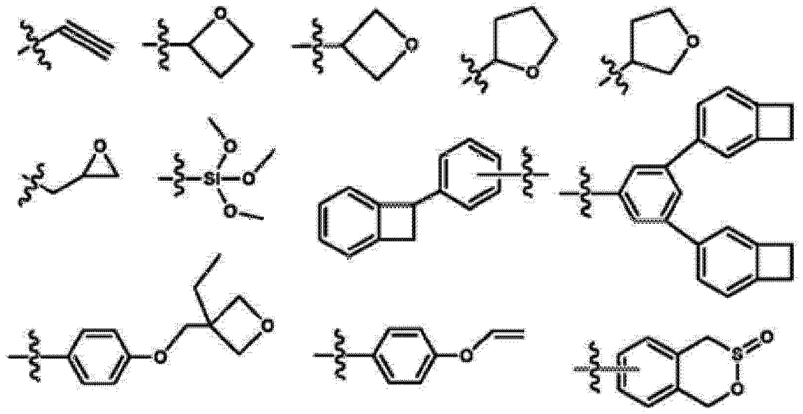

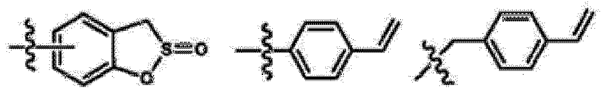

In the structures,
L11 is a direct bond; -O-; -S-; a substituted or unsubstituted alkylene group; a substituted or unsubstituted arylene group; or a substituted or unsubstituted heteroarylene group,
Lk is 1 or 2,
when lk is 2, L11s are the same as or different from each other, and
R21 is a substituted or unsubstituted alkyl group.

According to one embodiment of the present disclosure, L11 is a direct bond; a methylene group; or an ethylene group.

According to another embodiment, L11 is a direct bond.

According to one embodiment of the present disclosure, R21 is a methyl group; or an ethyl group.

According to another embodiment, R21 is a methyl group.

In the present disclosure, 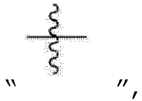 "*" and "-" each mean a linked site.

In the present disclosure, the radical compound means a compound having an unpaired electron. In addition, a radical compound means a compound carrying an unpaired electron, and depending on the type of the compound, a compound that is both a cation and a radical may exist. For reference, a cation compound in the present disclosure means a compound having a positive net charge by having more protons than electrons. A Π-radical compound is generally known to be unstable, however, a cation radical material may be stably present when having a proper structure such as a Π-conjugated system or an alkyl substituent.

In the present disclosure, a term "combination thereof" included in a Markush-type expression means a mixture or a combination of one or more selected from the group consisting of constituents described in the Markush-type expression, and means including one or more selected from the group consisting of the constituents.

Examples of substituents in the present disclosure are described below, however, the substituents are not limited thereto.

In the present disclosure, the term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present disclosure, the term "substituted or unsubstituted" means being substituted with one or more substituents selected from the group consisting of deuterium; a halogen group; a cyano group; an alkyl group; a cycloalkyl group; an alkoxy group; a silyl group; an aryl group; a curing group; and a heteroaryl group, or being substituted with a substituent linking two or more substituents among the substituents illustrated above, or having no substituents.

In the present disclosure, the halogen group is a fluoro group (-F), a chloro group (-Cl), a bromo group (-Br) or an iodo group (-I).

In the present disclosure, the alkyl group may be linear or branched, and although not particularly limited thereto, the number of carbon atoms may be from 1 to 20. According to another embodiment, the number of carbon atoms of the alkyl group is from 1 to 10. Specific examples of the alkyl group may include a methyl group, an ethyl group, an n-propyl group, an isopropyl group, an n-butyl group, an isobutyl group, a tert-butyl group, an n-pentyl group, an n-hexyl group, an n-heptyl group and the like, but are not limited thereto.

In the present disclosure, the cycloalkyl group is not particularly limited, but may have 3 to 60 carbon atoms, and according to one embodiment, the number of carbon atoms of the cycloalkyl group is from 3 to 30. According to another embodiment, the number of carbon atoms of the cycloalkyl group is from 3 to 20. Specific examples of the cycloalkyl group may include a cyclopropyl group, a cyclobutyl group, a cyclopentyl group, a cyclohexyl group, a cycloheptyl group, a cyclooctyl group and the like, but are not limited thereto.

In the present disclosure, the alkoxy group may be linear or branched. The number of carbon atoms of the alkoxy group is not particularly limited, but may be from 1 to 20. Specific examples of the alkoxy group may include a methoxy group, an ethoxy group, an n-propoxy group, an n-butoxy group, a tert-butoxy group, an n-pentyloxy group, an n-hexyloxy group, an n-octyloxy group, an n-nonyloxy group, an n-decyloxy group and the like, but are not limited thereto.

In the present disclosure, the aryloxy group means - OR_{aryloxy}, and R_{aryloxy} means an aryl group.

In the present disclosure, the silyl group means - SiRₛᵢₗ₁Rₛᵢₗ₂Rₛᵢₗ₃, and Rₛᵢₗ₁, Rₛᵢₗ₂ and Rₛᵢₗ₃ are the same as or different from each other and each independently hydrogen, deuterium, an alkyl group, a deuterated alkyl group, a fluoroalkyl group, an aryl group or a deuterated aryl group. When Rₛᵢₗ₁, Rₛᵢₗ₂ and Rₛᵢₗ₃ are each an alkyl group in some embodiments, one or more carbons in the alkyl group are replaced by Si.

In the present disclosure, the aryl group is not particularly limited, but may have 6 to 60 carbon atoms, and the aryl group may be a monocyclic aryl group or a polycyclic aryl group. According to one embodiment, the number of carbon atoms of the aryl group is from 6 to 30. According to one embodiment, the number of carbon atoms of the aryl group is from 6 to 20. When the aryl group is a monocyclic aryl group, examples thereof may include a phenyl group, a biphenyl group, a terphenyl group and the like, but are not limited thereto. When the aryl group is a polycyclic aryl group, examples thereof may include a naphthyl group, an anthracenyl group, a phenanthrenyl group, a pyrenyl group, a perylenyl group, a triphenylenyl group, a chrysenyl group, a fluorenyl group and the like, but are not limited thereto.

In the present disclosure, the fluorenyl group may be substituted, and two substituents may bond to each other to form a spiro structure.

When the fluorenyl group is substituted, a spirofluorenyl group such as 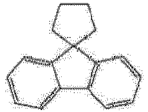 and 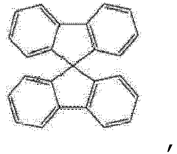 and a substituted fluorenyl group such as 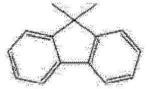 (9,9-dimethylfluorenyl group) and 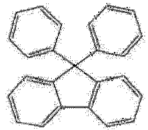 (9,9-diphenylfluorenyl group) may be included. However, the structure is not limited thereto.

In the present disclosure, the heteroaryl group is an aromatic cyclic group including one or more of N, O, P, S, Si and Se as a heteroatom, and although not particularly limited thereto, the number of carbon atoms may be from 2 to 60. According to one embodiment, the number of carbon atoms of the heteroaryl group is from 2 to 30. Examples of the heteroaryl group may include a pyridine group, a pyrrole group, a pyrimidine group, a pyridazine group, a furan group, a thiophene group, a benzothiophene group, a benzofuran group, a dibenzothiophene group, a dibenzofuran group and the like, but are not limited thereto.

In the present disclosure, the descriptions on the alkyl group provided above may be applied to the alkylene group except that the alkylene group is divalent.

In the present disclosure, the descriptions on the aryl group provided above may be applied to the arylene group except that the arylene group is divalent.

In the present disclosure, the descriptions on the heteroaryl group provided above may be applied to the heteroarylene group except that the heteroarylene group is divalent.

In the present disclosure, an aliphatic ring is a hydrocarbon ring that is not aromatic, and examples thereof may include the examples of the cycloalkyl group described above, an adamantly group and the like.

In the present disclosure, the descriptions on the aryl group provided above may be applied to the aromatic ring.

In the present disclosure, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

In the present disclosure, the "ring" in the substituted or unsubstituted ring formed by bonding to each other means a hydrocarbon ring; or a heteroring. The hydrocarbon ring may be aromatic, aliphatic, or a fused ring of aromatic and aliphatic. As for the heteroring, the descriptions on the heterocyclic group may be applied except for those that are divalent.

In the present disclosure, the descriptions on the aryl group provided above may be applied to the aromatic hydrocarbon ring except for those that are a divalent group.

In the present disclosure, the descriptions on the cycloalkyl group provided above may be applied to the aliphatic hydrocarbon ring except for those that are divalent.

In the present disclosure, a mole fraction means a ratio of the number of moles of a given component with respect to a total number of moles of all components.

One embodiment of the present disclosure provides a compound represented by the following Chemical Formula 1.

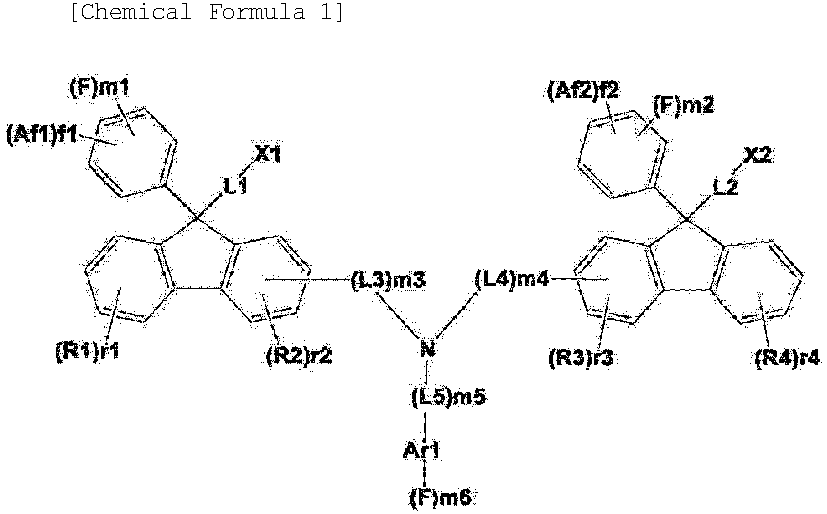

In Chemical Formula 1,
L1 and L2 are the same as or different from each other, and each independently a direct bond; a substituted or unsubstituted alkylene group; or a substituted or unsubstituted arylene group,
L3 to L5 are the same as or different from each other, and each independently a direct bond; or a substituted or unsubstituted arylene group,
R1 to R4 are the same as or different from each other, and each independently hydrogen; deuterium; a substituted or unsubstituted alkyl group; a substituted or unsubstituted alkoxy group; a substituted or unsubstituted aryl group; or a substituted or unsubstituted heteroaryl group,
Af1 and Af2 are the same as or different from each other, and each independently a substituted or unsubstituted alkyl group; or a substituted or unsubstituted aryl group,
Ar1 is a substituted or unsubstituted aryl group,
X1 and X2 are the same as or different from each other, and each independently -(R101)s; or -Y-A, and at least one of X1 and X2 is -Y-A,
R101 is hydrogen; deuterium; a halogen group; a substituted or unsubstituted alkyl group; a substituted or unsubstituted alkoxy group; a substituted or unsubstituted aryl group; or a substituted or unsubstituted aryloxy group,
s is an integer of 0 to 5, and when s is 2 or greater, the two or more R101s are the same as or different from each other,
Y is a direct bond, O or S,
A is a curing group,
r1 and r4 are each an integer of 0 to 4,
r2 and r3 are each an integer of 0 to 3,
m1+m2 is an integer of 2 to 10,
m3 to m5 are each an integer of 0 to 2,
m6 is an integer of 0 to 5,
f1 and f2 are each an integer of 0 to 5, f1+m1 is 5 or less, and f2+m2 is 5 or less,
when r1 to r4 are each 2 or greater, substituents in the parentheses are the same as or different from each other, and
when m3 to m5 are each 2, substituents in the parentheses are the same as or different from each other.

In the compound of Chemical Formula 1 according to one embodiment of the present disclosure, the number of nitrogen in the molecule is limited to one and Ar1 has a substituted or unsubstituted aryl group, which increases solubility for a solvent used in the process. In addition, the HOMO energy level is deepened. Accordingly, holes may readily pass from a hole injection layer to a hole transfer layer due to the HOMO energy level, and an increased device lifetime is obtained.

By the compound of Chemical Formula 1 according to one embodiment of the present disclosure including a fluoro group in the molecule, interfacial properties between a hole transfer layer and a hole injection layer are improved, and the HOMO energy level is deepened. Accordingly, holes readily migrate between the hole transfer layer and the hole injection layer, and excellent driving voltage and lifetime properties are obtained. Particularly, by the compound of Chemical Formula 1 according to one embodiment of the present disclosure being substituted with two or more fluoro groups (-F) in the molecule, interfacial properties between a hole injection layer and a hole transfer layer are improved, and by increasing stability of the fluoro group-substituted phenyl group, device properties are improved.

According to one embodiment of the present disclosure, L1 and L2 are the same as or different from each other, and each independently a direct bond; a substituted or unsubstituted alkylene group; or a substituted or unsubstituted arylene group. According to one embodiment of the present disclosure, L1 and L2 are the same as or different from each other, and each independently a direct bond; an alkylene group; or an arylene group.

According to one embodiment of the present disclosure, L1 and L2 are the same as or different from each other, and each independently a direct bond; a substituted or unsubstituted alkylene group having 1 to 20 carbon atoms; or a substituted or unsubstituted arylene group having 6 to 30 carbon atoms.

According to one embodiment of the present disclosure, L1 and L2 are the same as or different from each other, and each independently a direct bond; a substituted or unsubstituted alkylene group having 1 to 10 carbon atoms; or a substituted or unsubstituted arylene group having 6 to 20 carbon atoms.

According to one embodiment of the present disclosure, L1 and L2 are the same as or different from each other, and each independently a direct bond; a substituted or unsubstituted alkylene group having 1 to 6 carbon atoms; or a substituted or unsubstituted arylene group having 6 to 15 carbon atoms.

According to one embodiment of the present disclosure, L1 and L2 are the same as or different from each other, and each independently a direct bond; a substituted or unsubstituted butylene group; a substituted or unsubstituted phenylene group; or a substituted or unsubstituted biphenylene group.

According to one embodiment of the present disclosure, L1 and L2 are the same as or different from each other, and each independently a direct bond; a butylene group; a phenylene group; or a biphenylene group.

According to one embodiment of the present disclosure, L3 and L4 are the same as or different from each other, and each independently a direct bond; or a substituted or unsubstituted arylene group.

According to one embodiment of the present disclosure, L3 and L4 are the same as or different from each other, and each independently a direct bond; or an arylene group.

According to one embodiment of the present disclosure, L3 and L4 are the same as or different from each other, and each independently a direct bond; or a substituted or unsubstituted arylene group having 6 to 30 carbon atoms.

According to one embodiment of the present disclosure, L3 and L4 are the same as or different from each other, and each independently a direct bond; or a substituted or unsubstituted arylene group having 6 to 20 carbon atoms.

According to one embodiment of the present disclosure, L3 and L4 are the same as or different from each other, and each independently a direct bond; or a substituted or unsubstituted arylene group having 6 to 15 carbon atoms.

According to one embodiment of the present disclosure, L3 and L4 are a direct bond.

According to one embodiment of the present disclosure, L5s are the same as or different from each other, and each independently a direct bond; or a substituted or unsubstituted arylene group having 6 to 30 carbon atoms.

According to one embodiment of the present disclosure, L5s are the same as or different from each other, and each independently a direct bond; or a substituted or unsubstituted arylene group having 6 to 20 carbon atoms.

According to one embodiment of the present disclosure, L5s are the same as or different from each other, and each independently a direct bond; or a substituted or unsubstituted arylene group having 6 to 18 carbon atoms.

According to one embodiment of the present disclosure, L5 is a direct bond; a substituted or unsubstituted phenylene group; a substituted or unsubstituted biphenylene group; or a substituted or unsubstituted terphenylene group.

According to one embodiment of the present disclosure, L5 is a direct bond; a phenylene group; a biphenylene group; or a terphenylene group.

According to one embodiment of the present disclosure, R1 to R4 are the same as or different from each other, and hydrogen; deuterium; a substituted or unsubstituted alkyl group; a substituted or unsubstituted alkoxy group; a substituted or unsubstituted aryl group; or a substituted or unsubstituted heteroaryl group.

According to one embodiment of the present disclosure, R1 to R4 are the same as or different from each other, and hydrogen; deuterium; an alkyl group; an alkoxy group; an aryl group; or a heteroaryl group.

According to one embodiment of the present disclosure, R1 to R4 are the same as or different from each other, and hydrogen; deuterium; a substituted or unsubstituted alkyl group having 1 to 20 carbon atoms; a substituted or unsubstituted alkoxy group having 1 to 20 carbon atoms; a substituted or unsubstituted aryl group having 6 to 30 carbon atoms; or a substituted or unsubstituted heteroaryl group having 2 to 30 carbon atoms.

According to one embodiment of the present disclosure, R1 to R4 are the same as or different from each other, and hydrogen; deuterium; a substituted or unsubstituted alkyl group having 1 to 10 carbon atoms; a substituted or unsubstituted alkoxy group having 1 to 10 carbon atoms; a substituted or unsubstituted aryl group having 6 to 20 carbon atoms; or a substituted or unsubstituted heteroaryl group having 3 to 20 carbon atoms.

According to one embodiment of the present disclosure, R1 to R4 are the same as or different from each other, and hydrogen; deuterium; a substituted or unsubstituted alkyl group having 1 to 6 carbon atoms; a substituted or unsubstituted alkoxy group having 1 to 6 carbon atoms; a substituted or unsubstituted aryl group having 6 to 15 carbon atoms; or a substituted or unsubstituted heteroaryl group having 3 to 15 carbon atoms.

According to one embodiment of the present disclosure, R1 to R4 are the same as or different from each other, and hydrogen or deuterium.

According to one embodiment of the present disclosure, R1 to R4 are hydrogen.

According to one embodiment of the present disclosure, Af1 and Af2 are the same as or different from each other, and each independently a substituted or unsubstituted alkyl group; or a substituted or unsubstituted aryl group.

According to one embodiment of the present disclosure, Af1 and Af2 are the same as or different from each other, and each independently an alkyl group; or an aryl group.

According to one embodiment of the present disclosure, Af1 and Af2 are the same as or different from each other, and each independently a substituted or unsubstituted alkyl group having 1 to 30 carbon atoms; or a substituted or unsubstituted aryl group having 6 to 30 carbon atoms.

According to one embodiment of the present disclosure, Af1 and Af2 are the same as or different from each other, and each independently a substituted or unsubstituted alkyl group having 1 to 20 carbon atoms; or a substituted or unsubstituted aryl group having 6 to 20 carbon atoms.

According to one embodiment of the present disclosure, Af1 and Af2 are the same as or different from each other, and each independently a substituted or unsubstituted alkyl group having 1 to 10 carbon atoms; or a substituted or unsubstituted aryl group having 6 to 18 carbon atoms.

According to one embodiment of the present disclosure, Af1 and Af2 are the same as or different from each other, and each independently a substituted or unsubstituted alkyl group having 1 to 4 carbon atoms; or a substituted or unsubstituted aryl group having 6 to 10 carbon atoms.

According to one embodiment of the present disclosure, Af1 and Af2 are the same as or different from each other, and each independently a substituted or unsubstituted methyl group; or a substituted or unsubstituted phenyl group.

According to one embodiment of the present disclosure, Af1 and Af2 are the same as or different from each other, and each independently a methyl group; or a phenyl group.

According to one embodiment of the present disclosure, Ar1 is a substituted or unsubstituted aryl group.

According to another embodiment of the present disclosure, Ar1 is an aryl group.

According to one embodiment of the present disclosure, Ar1 is a substituted or unsubstituted aryl group having 6 to 30 carbon atoms.

According to one embodiment of the present disclosure, Ar1 is a substituted or unsubstituted aryl group having 6 to 20 carbon atoms.

According to one embodiment of the present disclosure, Ar1 is a substituted or unsubstituted aryl group having 6 to 18 carbon atoms.

According to one embodiment of the present disclosure, Ar1 is a substituted or unsubstituted phenyl group; a substituted or unsubstituted biphenyl group; or a substituted or unsubstituted terphenyl group.

According to one embodiment of the present disclosure, Ar1 is a phenyl group; a biphenyl group; or a terphenyl group.

According to one embodiment of the present disclosure, X1 and X2 are the same as or different from each other and each independently -(R101)s; or -Y-A, and at least one of X1 and X2 is -Y-A.

According to one embodiment of the present disclosure, R101 is hydrogen; deuterium; a halogen group; a substituted or unsubstituted alkyl group; a substituted or unsubstituted alkoxy group; a substituted or unsubstituted aryl group; or a substituted or unsubstituted aryloxy group.

According to one embodiment of the present disclosure, R101 is hydrogen; deuterium; a halogen group; an alkyl group unsubstituted or substituted with an alkyl group; an alkoxy group unsubstituted or substituted with an alkyl group; an aryl group unsubstituted or substituted with an alkyl group; or an aryloxy group unsubstituted or substituted with an alkyl group.

According to one embodiment of the present disclosure, R101 is hydrogen; deuterium; a halogen group; an alkyl group; an alkoxy group; an aryl group; or an aryloxy group.

According to one embodiment of the present disclosure, R101 is hydrogen; deuterium; a halogen group; a substituted or unsubstituted alkyl group having 1 to 20 carbon atoms; a substituted or unsubstituted alkoxy group having 1 to 20 carbon atoms; a substituted or unsubstituted aryl group having 6 to 30 carbon atoms; or a substituted or unsubstituted aryloxy group having 6 to 30 carbon atoms.

According to one embodiment of the present disclosure, R101 is hydrogen; deuterium; a halogen group; a substituted or unsubstituted alkyl group having 1 to 10 carbon atoms; a substituted or unsubstituted alkoxy group having 1 to 10 carbon atoms; a substituted or unsubstituted aryl group having 6 to 20 carbon atoms; or a substituted or unsubstituted aryloxy group having 6 to 20 carbon atoms.

According to one embodiment of the present disclosure, R101 is hydrogen; deuterium; a halogen group; a substituted or unsubstituted alkyl group having 1 to 6 carbon atoms; a substituted or unsubstituted alkoxy group having 1 to 6 carbon atoms; a substituted or unsubstituted aryl group having 6 to 18 carbon atoms; or a substituted or unsubstituted aryloxy group having 6 to 18 carbon atoms.

According to one embodiment of the present disclosure, R101 is hydrogen; deuterium; a halogen group; a substituted or unsubstituted phenyl group; or a substituted or unsubstituted biphenyl group.

According to one embodiment of the present disclosure, R101 is hydrogen; a substituted or unsubstituted phenyl group; or a substituted or unsubstituted biphenyl group.

According to one embodiment of the present disclosure, R101 is a substituted or unsubstituted phenyl group; or a substituted or unsubstituted biphenyl group.

According to one embodiment of the present disclosure, R101 is a phenyl group unsubstituted or substituted with an alkyl group; or a biphenyl group unsubstituted or substituted with an alkyl group.

According to one embodiment of the present disclosure, R101 is a phenyl group unsubstituted or substituted with an ethyl group; or a biphenyl group unsubstituted or substituted with an ethyl group.

According to one embodiment of the present disclosure, Y is a direct bond, O or S.

According to one embodiment of the present disclosure, A is a curing group.

According to one embodiment of the present disclosure, A is any one selected from among the following structures.

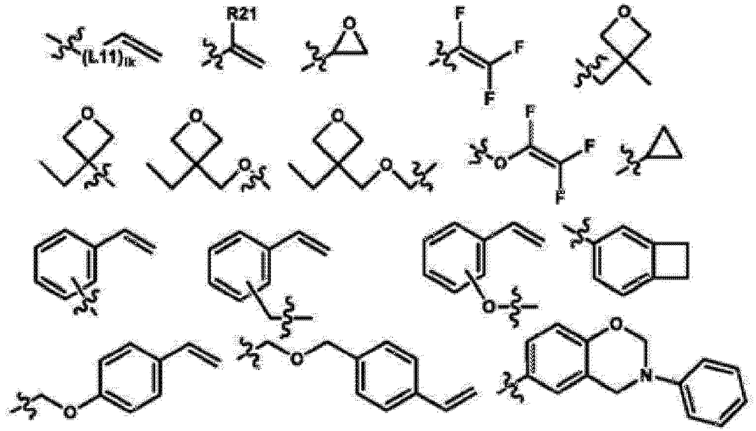

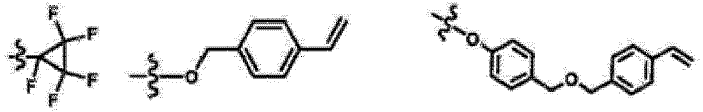

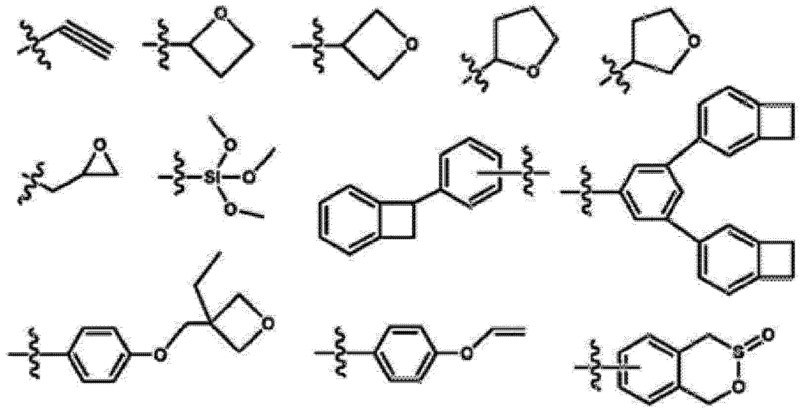

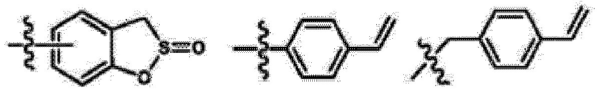

In the structures, L11 and R21 have the same definitions as in the curing group.

According to one embodiment of the present disclosure, A may be selected from among the following structures.

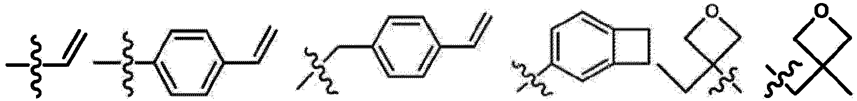

According to one embodiment of the present disclosure, m1+m2 is an integer of 2 to 10. According to another embodiment of the present disclosure, m1+m2 is an integer of 2. According to another embodiment of the present disclosure, m1+m2 is an integer of 3. According to another embodiment of the present disclosure, m1+m2 is an integer of 4. According to another embodiment of the present disclosure, m1+m2 is an integer of 5. According to another embodiment of the present disclosure, m1+m2 is an integer of 6. According to another embodiment of the present disclosure, m1+m2 is an integer of 7. According to another embodiment of the present disclosure, m1+m2 is an integer of 8. According to another embodiment of the present disclosure, m1+m2 is an integer of 9. According to another embodiment of the present disclosure, m1+m2 is an integer of 10.

According to one embodiment of the present disclosure, m1 and m2 are each an integer of 1 to 5.

According to one embodiment of the present disclosure, m1 is an integer of 1 to 5. According to another embodiment of the present disclosure, m1 is an integer of 1. According to another embodiment of the present disclosure, m1 is an integer of 2. According to another embodiment of the present disclosure, m1 is an integer of 3. According to another embodiment of the present disclosure, m1 is an integer of 4. According to another embodiment of the present disclosure, m1 is an integer of 5.

According to one embodiment of the present disclosure, m2 is an integer of 1 to 5. According to another embodiment of the present disclosure, m2 is an integer of 1. According to another embodiment of the present disclosure, m2 is an integer of 2. According to another embodiment of the present disclosure, m2 is an integer of 3. According to another embodiment of the present disclosure, m2 is an integer of 4. According to another embodiment of the present disclosure, m2 is an integer of 5.

According to one embodiment of the present disclosure, m1 and m2 are an integer of 1. According to another embodiment of the present disclosure, m1 and m2 are an integer of 2. According to another embodiment of the present disclosure, m1 and m2 are an integer of 3. According to another embodiment of the present disclosure, m1 and m2 are an integer of 4. According to another embodiment of the present disclosure, m1 and m2 are an integer of 5.

According to one embodiment of the present disclosure, m3 is an integer of 0 to 2. According to another embodiment of the present disclosure, m3 is an integer of 0. According to another embodiment of the present disclosure, m3 is an integer of 1. According to another embodiment of the present disclosure, m3 is an integer of 2. When m3 is 2, L3s are the same as or different from each other.

According to one embodiment of the present disclosure, m4 is an integer of 0 to 2. According to another embodiment of the present disclosure, m4 is an integer of 0. According to another embodiment of the present disclosure, m4 is an integer of 1. According to another embodiment of the present disclosure, m4 is an integer of 2. When m4 is 2, L4s are the same as or different from each other.

According to one embodiment of the present disclosure, m5 is an integer of 0 to 2. According to another embodiment of the present disclosure, m5 is an integer of 0. According to another embodiment of the present disclosure, m5 is an integer of 1. According to another embodiment of the present disclosure, m5 is an integer of 2. When m5 is 2, L5s are the same as or different from each other.

According to one embodiment of the present disclosure, m6 is an integer of 0 to 5. According to another embodiment of the present disclosure, m6 is an integer of 0. According to another embodiment of the present disclosure, m6 is an integer of 1. According to another embodiment of the present disclosure, m6 is an integer of 2. According to another embodiment of the present disclosure, m6 is an integer of 3. According to another embodiment of the present disclosure, m6 is an integer of 4. According to another embodiment of the present disclosure, m6 is an integer of 5.

According to one embodiment of the present disclosure, s is an integer of 0 to 5. According to another embodiment of the present disclosure, s is an integer of 1. According to another embodiment of the present disclosure, s is an integer of 2. According to another embodiment of the present disclosure, s is an integer of 3. According to another embodiment of the present disclosure, s is an integer of 4. According to another embodiment of the present disclosure, s is an integer of 5. When s is 2 or greater, the two or more R101s are the same as or different from each other.

According to one embodiment of the present disclosure, r1 and r4 are each an integer of 0 to 4.

According to one embodiment of the present disclosure, r1 is an integer of 0 to 4. According to another embodiment of the present disclosure, r1 is an integer of 0. According to another embodiment of the present disclosure, r1 is an integer of 1. According to another embodiment of the present disclosure, r1 is an integer of 2. According to another embodiment of the present disclosure, r1 is an integer of 3. According to another embodiment of the present disclosure, r1 is an integer of 4.

According to one embodiment of the present disclosure, r4 is an integer of 0 to 4. According to another embodiment of the present disclosure, r4 is an integer of 0. According to another embodiment of the present disclosure, r4 is an integer of 1. According to another embodiment of the present disclosure, r4 is an integer of 2. According to another embodiment of the present disclosure, r4 is an integer of 3. According to another embodiment of the present disclosure, r4 is an integer of 4.

According to one embodiment of the present disclosure, r2 and r3 are each an integer of 0 to 3.

According to one embodiment of the present disclosure, r2 is an integer of 0 to 3. According to another embodiment of the present disclosure, r2 is an integer of 0. According to another embodiment of the present disclosure, r2 is an integer of 1. According to another embodiment of the present disclosure, r2 is an integer of 2. According to another embodiment of the present disclosure, r2 is an integer of 3.

According to one embodiment of the present disclosure, r3 is an integer of 0 to 3. According to another embodiment of the present disclosure, r3 is an integer of 0. According to another embodiment of the present disclosure, r3 is an integer of 1. According to another embodiment of the present disclosure, r3 is an integer of 2. According to another embodiment of the present disclosure, r3 is an integer of 3.

When r1 to r4 are 2 or greater, substituents in the parentheses are the same as or different from each other.

According to one embodiment of the present disclosure, f1 and f2 are each an integer of 0 to 5.

According to one embodiment of the present disclosure, f1 is an integer of 0 to 5. According to another embodiment of the present disclosure, f1 is an integer of 0. According to another embodiment of the present disclosure, f1 is an integer of 1. According to another embodiment of the present disclosure, f1 is an integer of 2. According to another embodiment of the present disclosure, f1 is an integer of 3. According to another embodiment of the present disclosure, f1 is an integer of 4. According to another embodiment of the present disclosure, f1 is an integer of 5.

According to one embodiment of the present disclosure, f2 is an integer of 0 to 5. According to another embodiment of the present disclosure, f2 is an integer of 0. According to another embodiment of the present disclosure, f2 is an integer of 1. According to another embodiment of the present disclosure, f2 is an integer of 2. According to another embodiment of the present disclosure, f2 is an integer of 3. According to another embodiment of the present disclosure, f2 is an integer of 4. According to another embodiment of the present disclosure, f2 is an integer of 5.

According to one embodiment of the present disclosure, f1+m1 is 5 or less.

According to one embodiment of the present disclosure, f2+m2 is 5 or less.

According to one embodiment of the present disclosure, Chemical Formula 1 is represented by the following Chemical Formula 2.

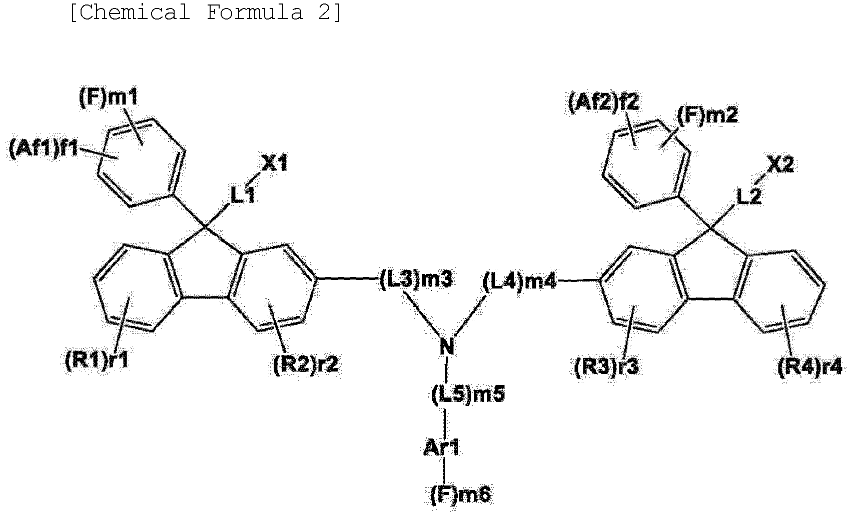

In Chemical Formula 2,
L1 to L5, R1 to R4, Af1, Af2, Ar1, X1, X2, r1 to r4, m1 to m6, f1 and f2 have the same definitions as in Chemical Formula 1.
According to one embodiment of the present disclosure, Chemical Formula 1 is represented by any one of the following Chemical Formulae 31 to 33.

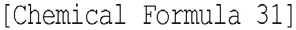

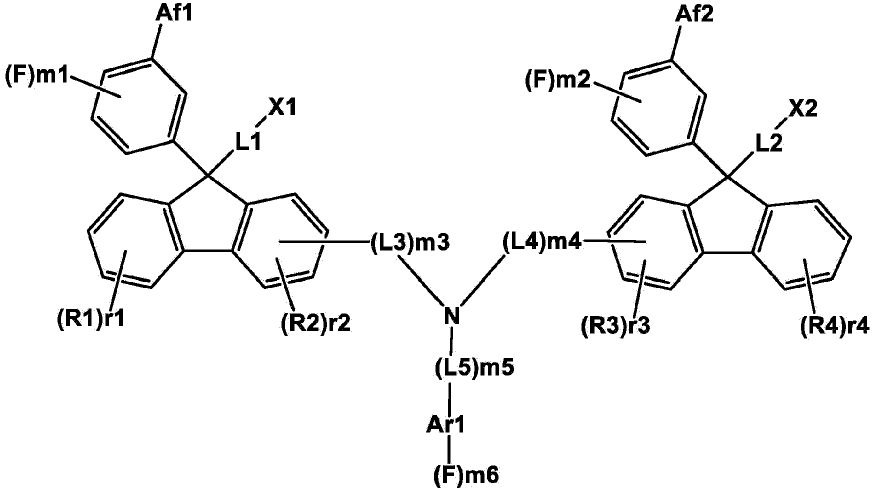

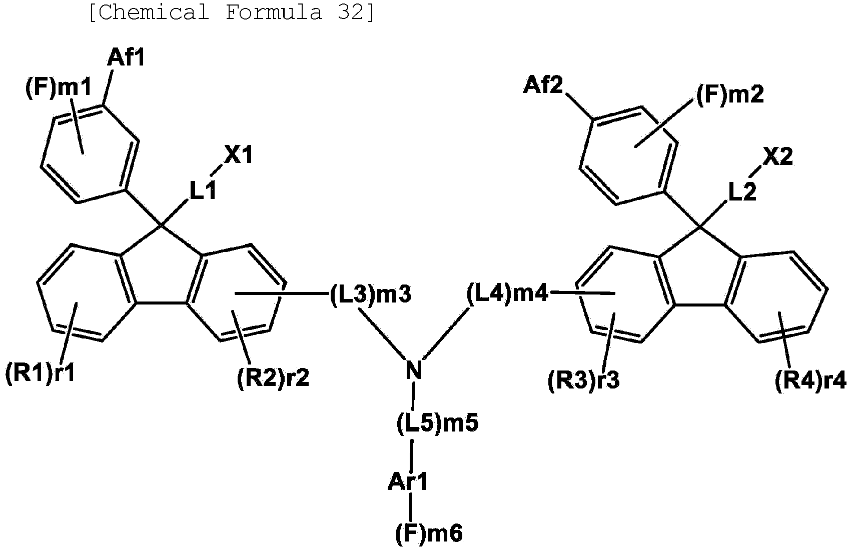

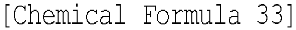

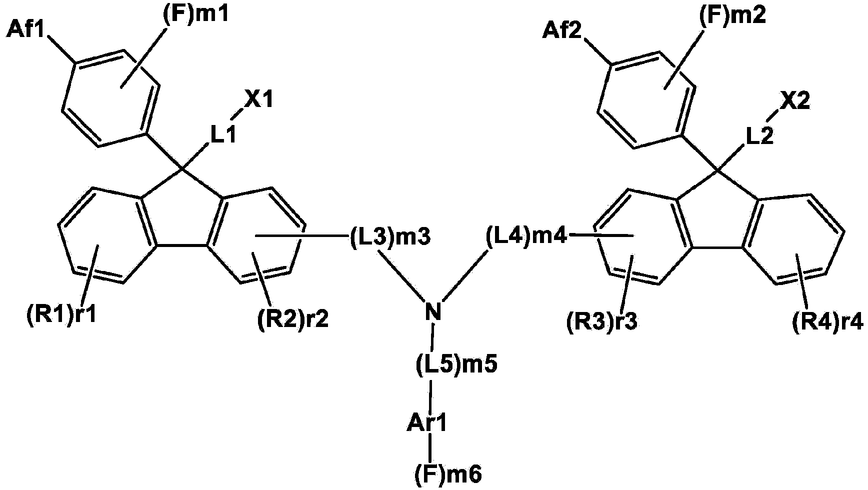

In Chemical Formulae 31 to 33, L1 to L5, R1 to R4, Af1, Af2, Ar1, X1, X2, r1 to r4 and m1 to m6 have the same definitions as in Chemical Formula 1.

According to one example on Chemical Formulae 31 to 33, m1 and m2 are 1. Specifically, according to one embodiment of the present disclosure, Chemical Formula 1 is represented by any one of the following Chemical Formulae 301 to 303.

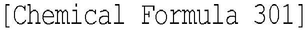

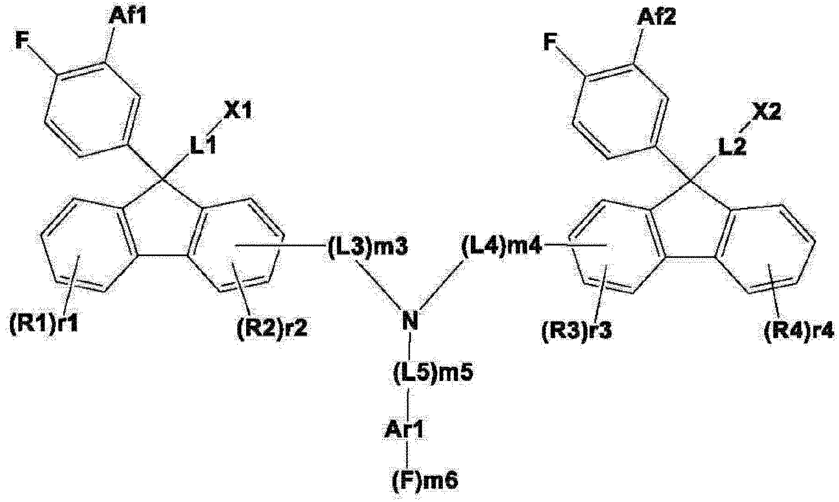

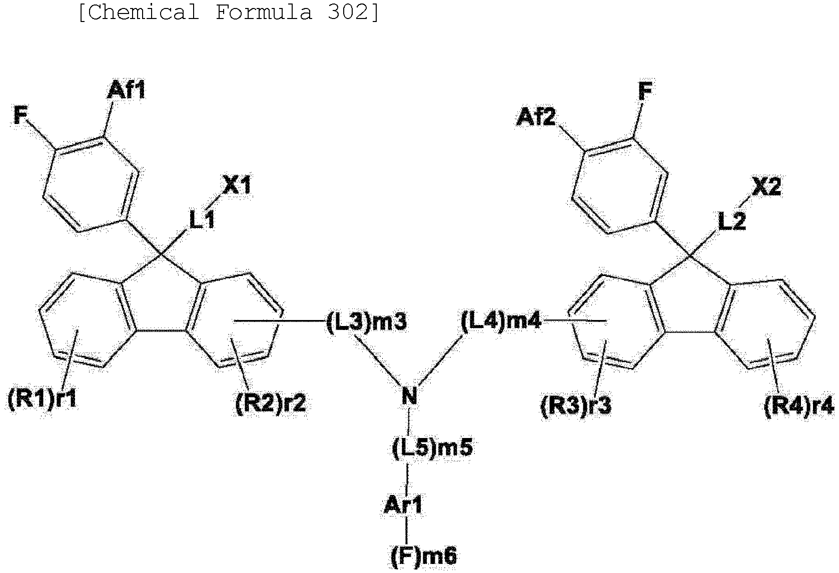

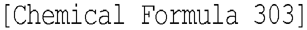

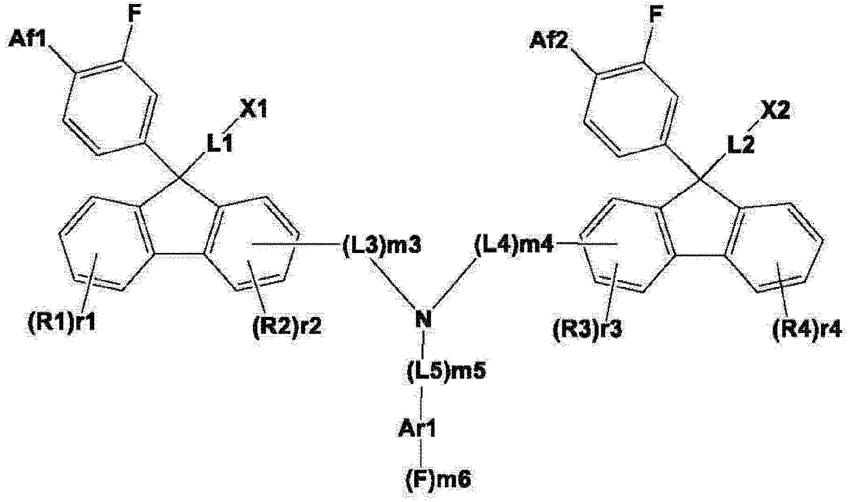

In Chemical Formulae 301 to 303, L1 to L5, R1 to R4, Af1, Af2, Ar1, X1, X2, r1 to r4 and m3 to m6 have the same definitions as in Chemical Formula 1.

Chemical Formulae 301 to 303 are cases in which f1, f2, m1 and m2 defined in Chemical Formula 1 are each 1.

The compound according to any one of Chemical Formulae 301 to 303 according to one embodiment of the present disclosure is effective in improving properties of an organic light emitting device by including one fluoro group in each phenyl group that substitutes fluorenyl groups bonding to left and right sides of the amine group, and including a substituted or unsubstituted alkyl group; or a substituted or unsubstituted aryl group at a specific position around the fluoro group. Specifically, the fluoro group-substituted phenyl group has increased stability in the compound in which Af1 or Af2 and a fluoro group bond to a meta or para position of the phenyl group compared to in the compound in which Af1 or Af2 and a fluoro group bond to an ortho position of the phenyl group, and accordingly, an organic light emitting device including the compound according to any one of Chemical Formulae 301 to 303 may have improved properties.

According to one embodiment of the present disclosure, Chemical Formula 1 is represented by any one of the following Chemical Formulae 41 to 43.

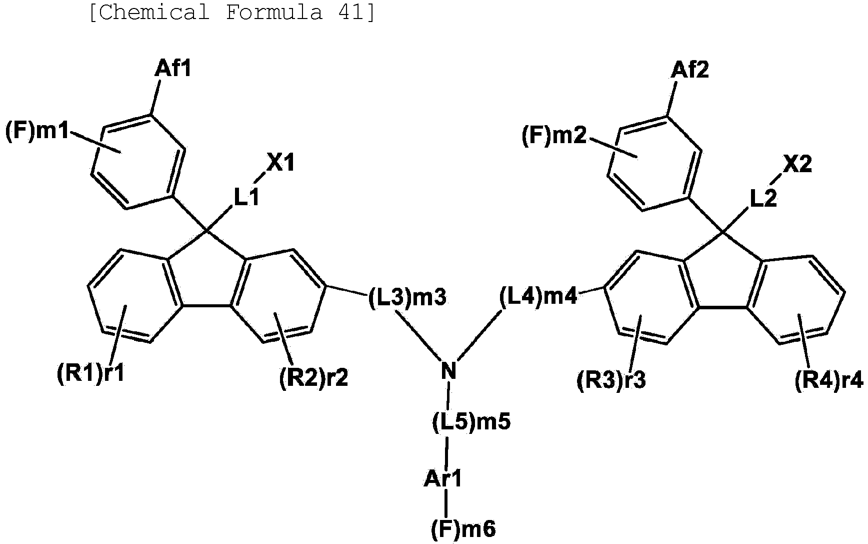

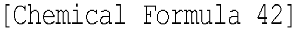

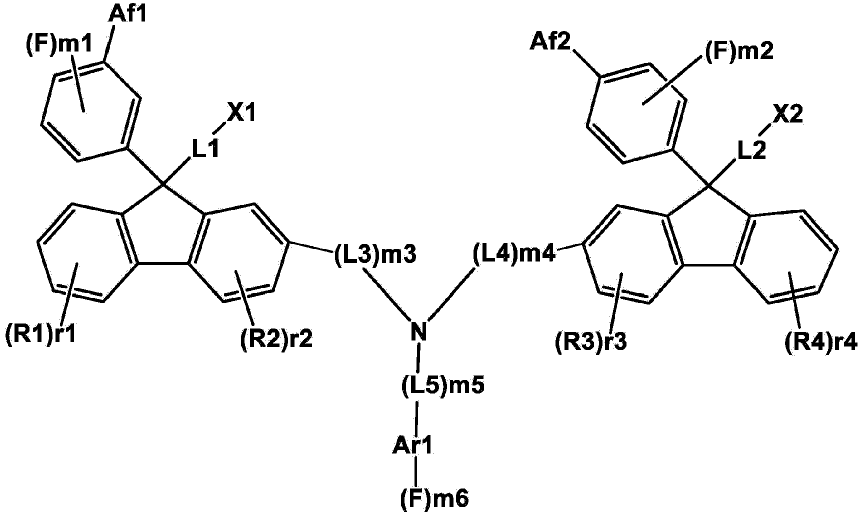

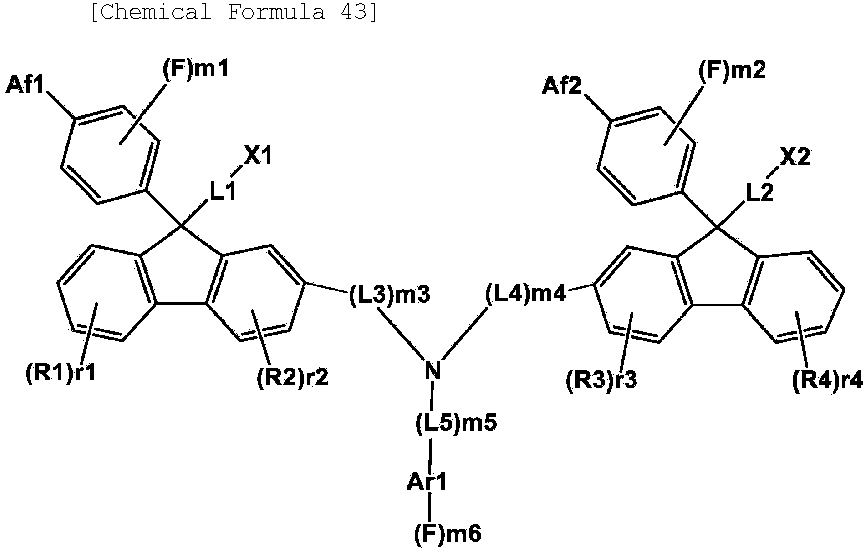

In Chemical Formulae 41 to 43, L1 to L5, R1 to R4, Af1, Af2, Ar1, X1, X2, r1 to r4 and m3 to m6 have the same definitions as in Chemical Formula 1.

According to one example on Chemical Formulae 41 to 43, m1 and m2 are 1. Specifically, according to one embodiment of the present disclosure, Chemical Formula 1 is represented by any one of the following Chemical Formulae 401 to 403.

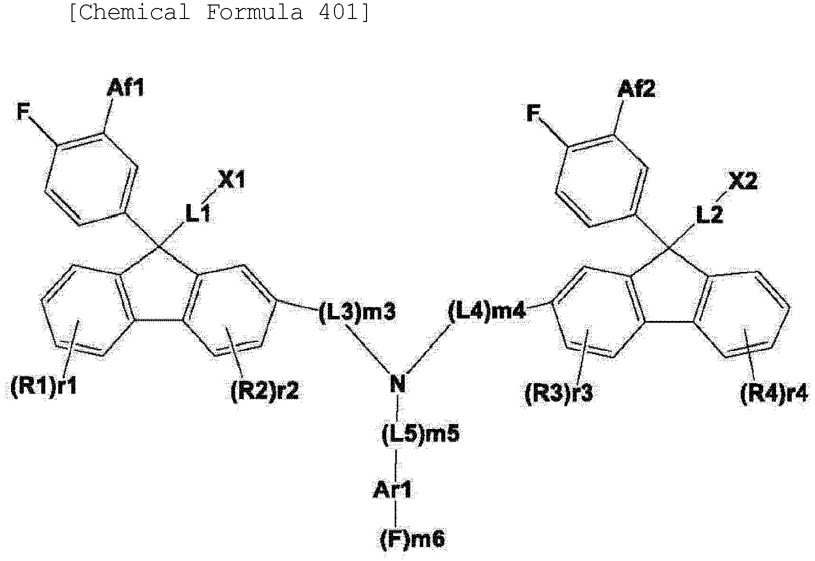

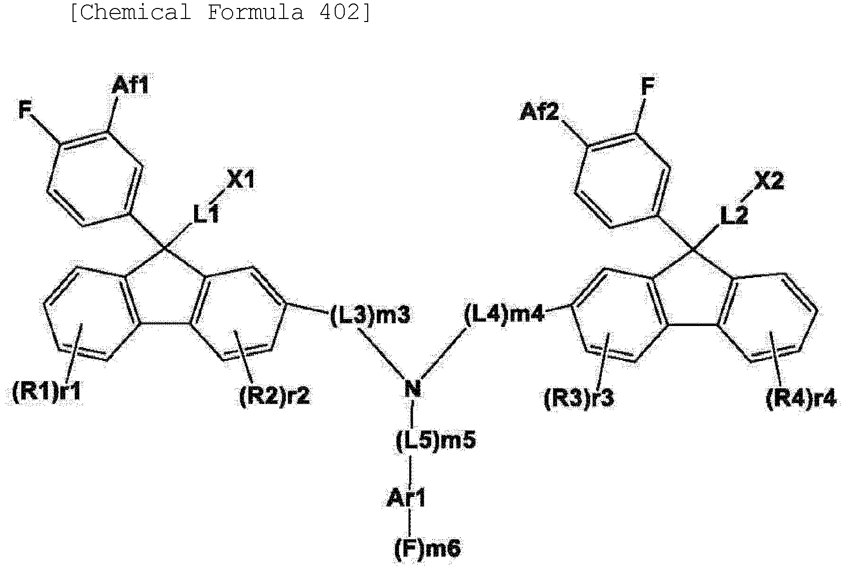

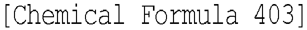

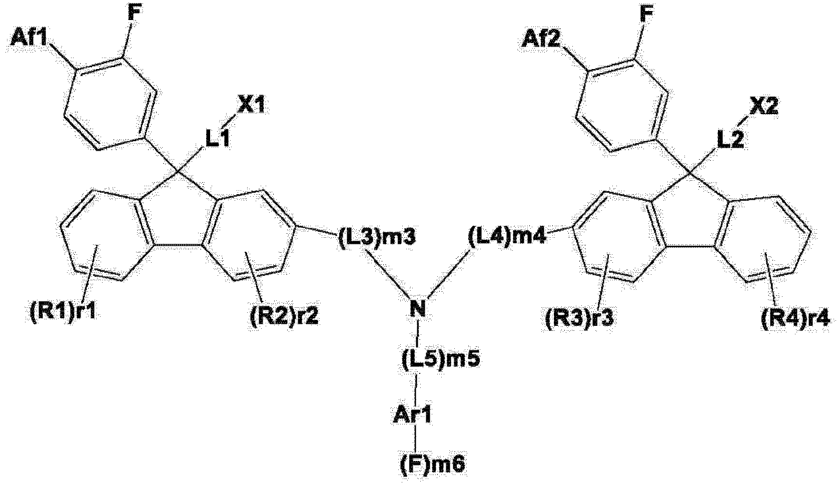

In Chemical Formulae 401 to 403, L1 to L5, R1 to R4, Af1, Af2, Ar1, X1, X2, r1 to r4 and m3 to m6 have the same definitions as in Chemical Formula 1.

Chemical Formulae 401 to 403 are cases in which m1, m2, f1 and f2 defined in Chemical Formula 1 are each 1.

According to one embodiment of the present disclosure, Chemical Formula 1 is any one selected from the group consisting of the following compounds.

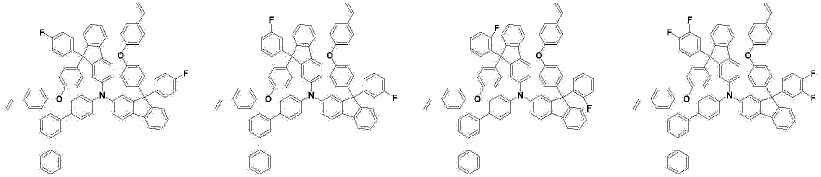

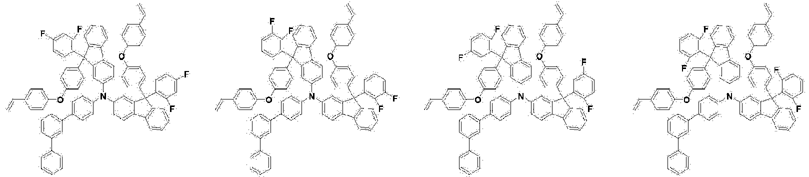

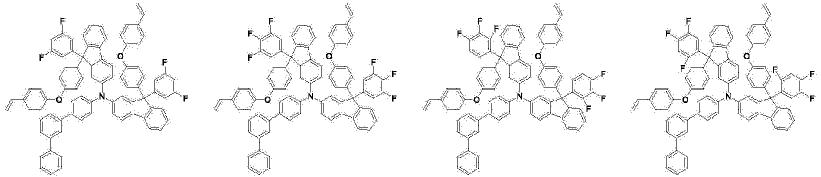

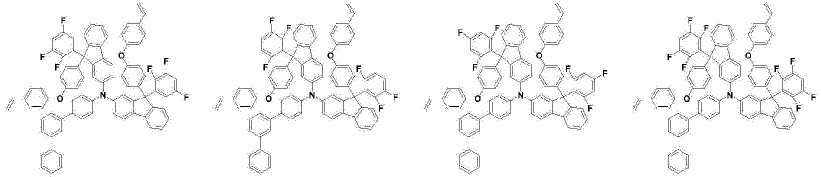

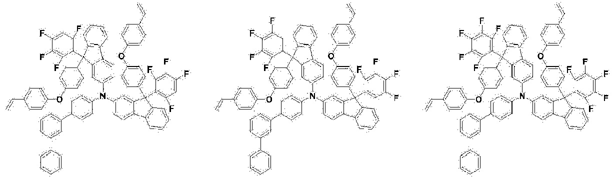

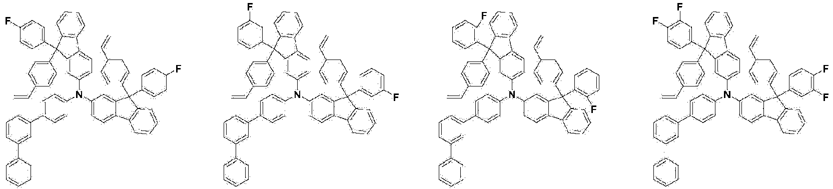

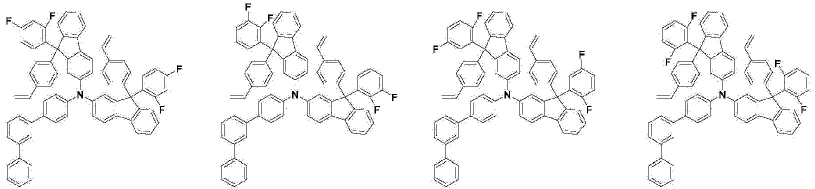

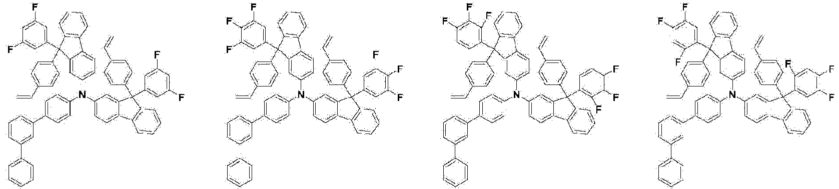

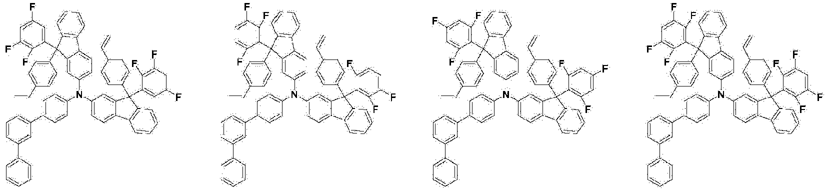

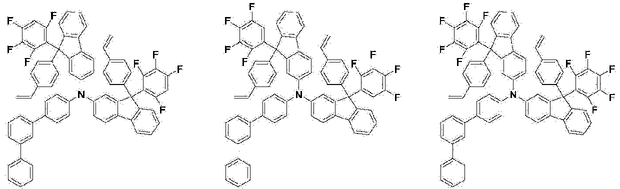

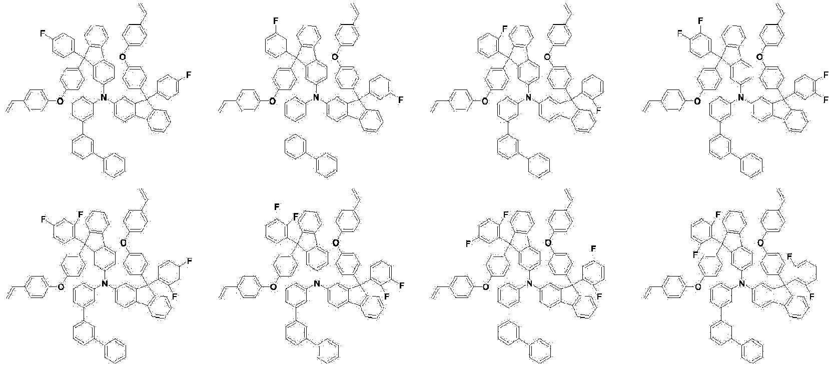

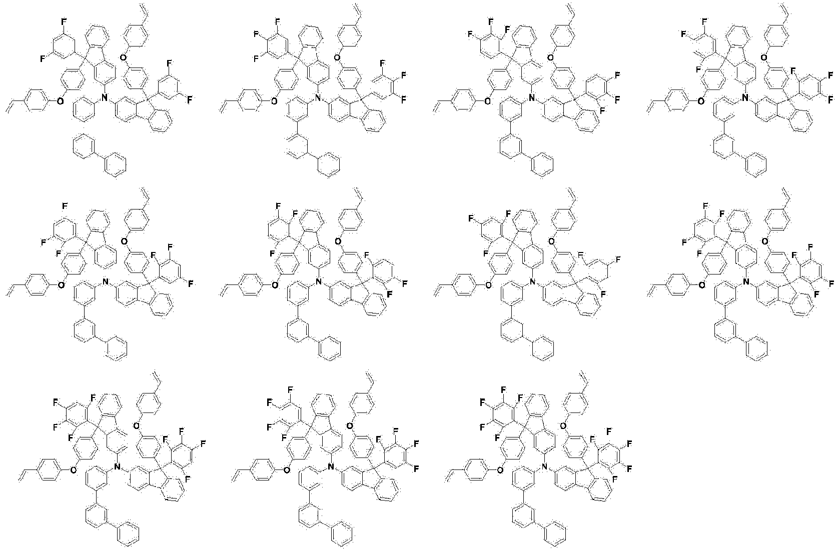

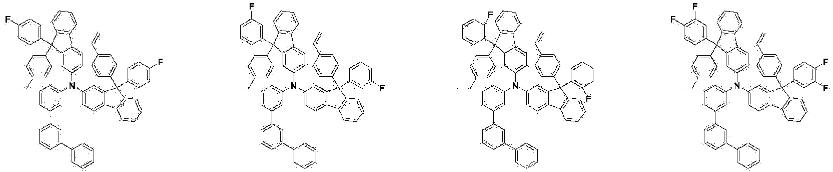

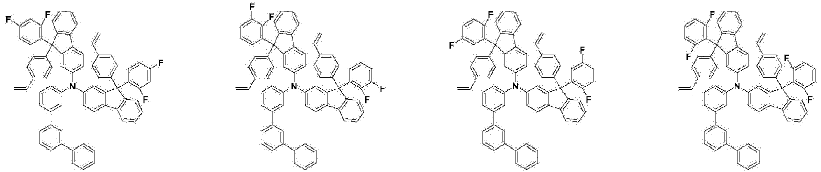

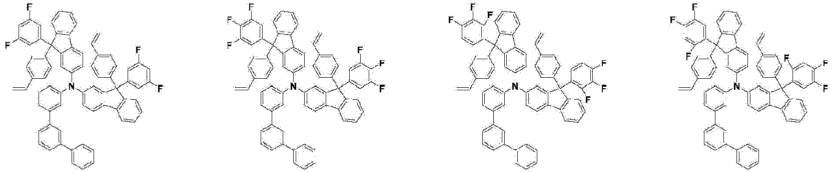

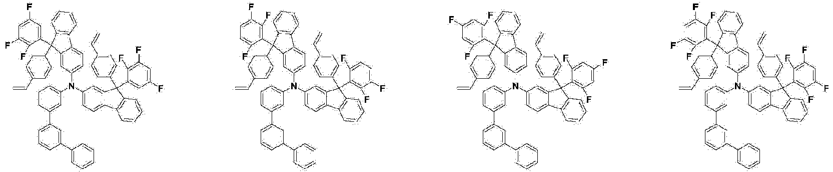

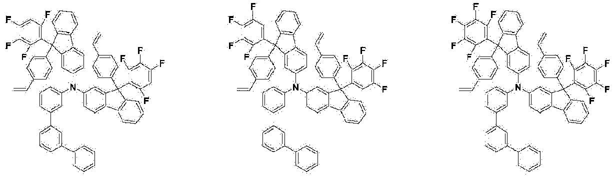

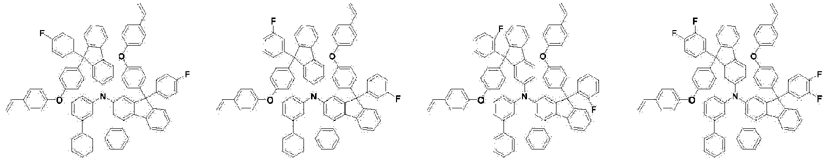

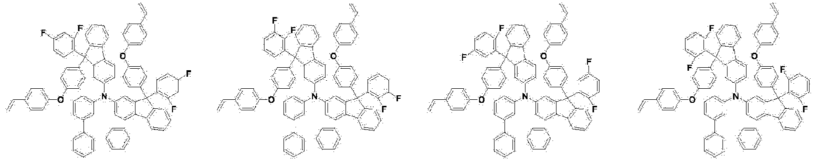

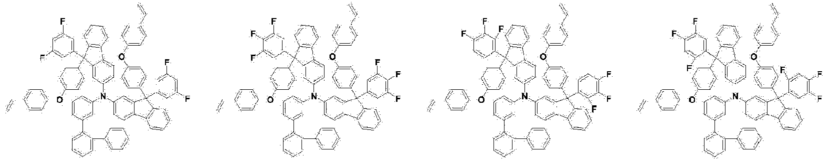

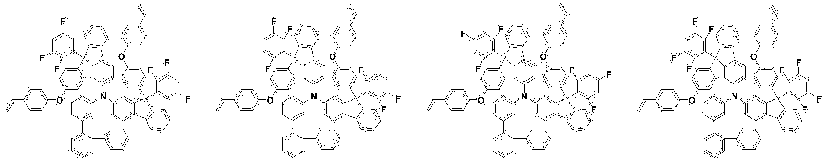

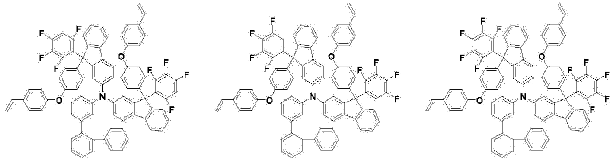

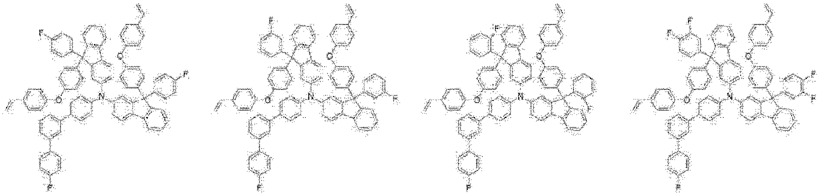

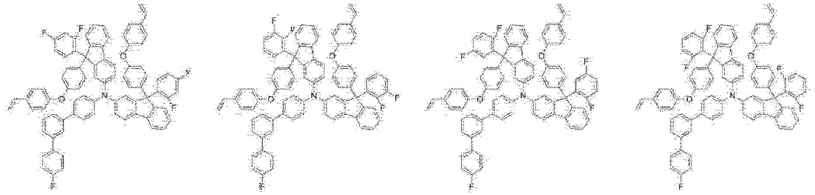

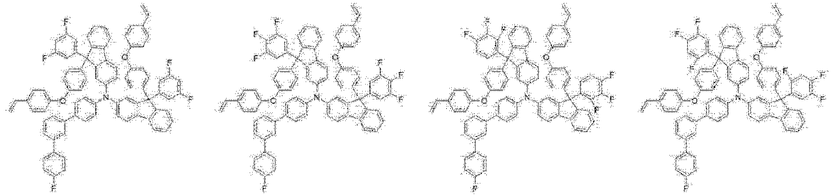

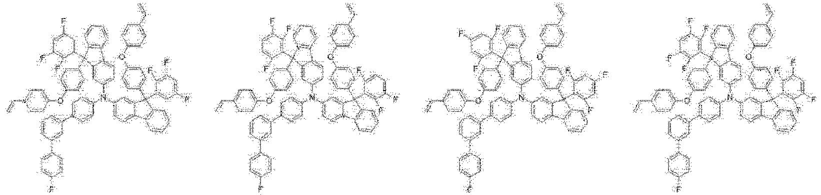

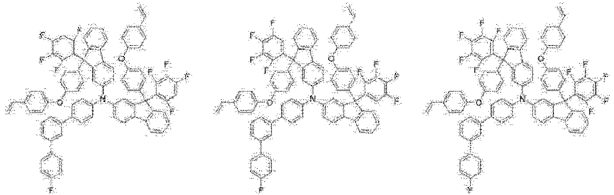

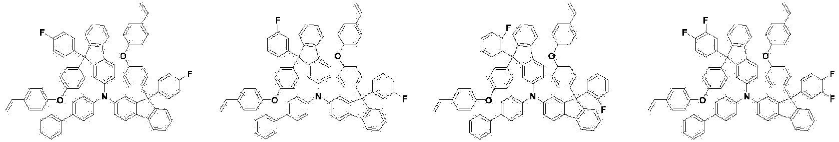

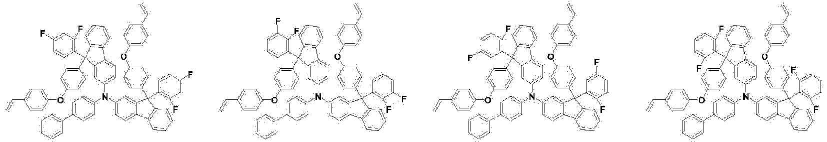

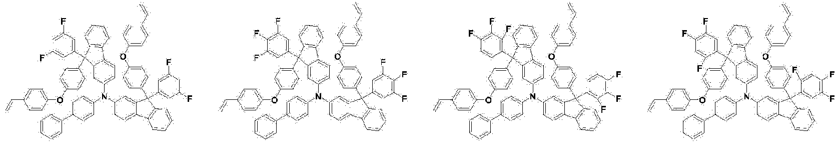

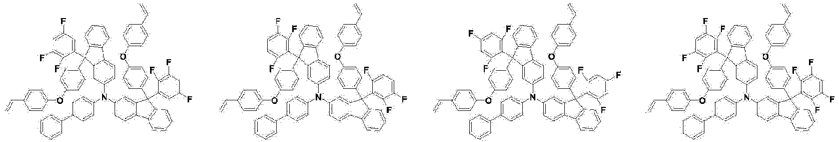

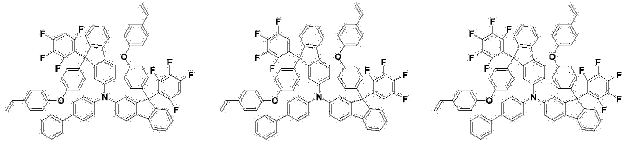

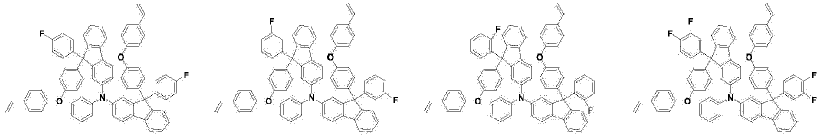

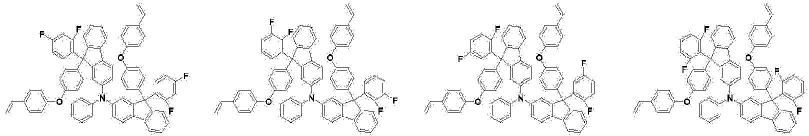

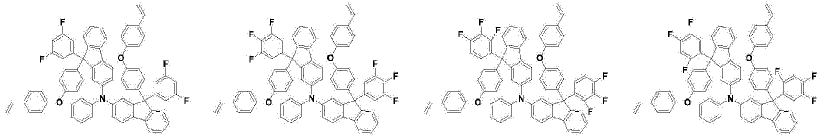

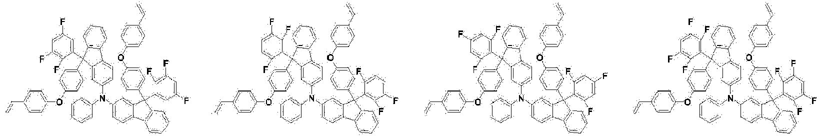

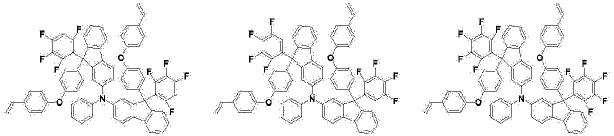

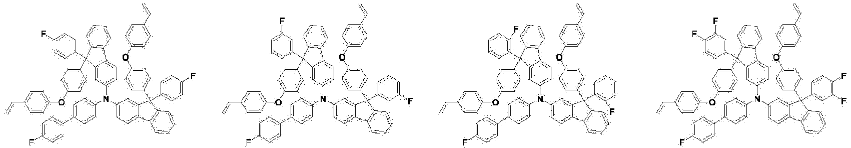

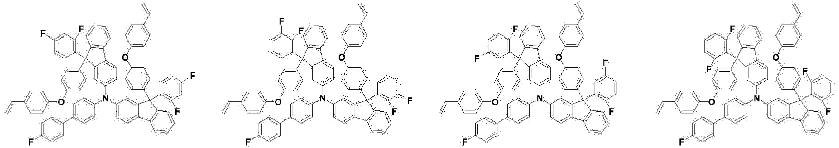

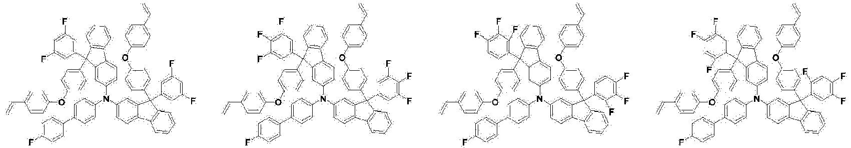

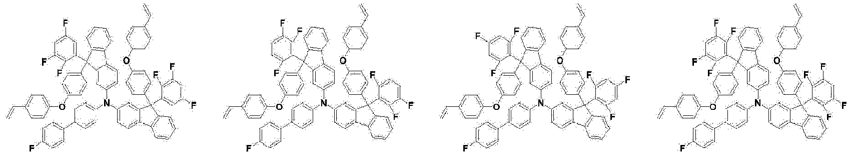

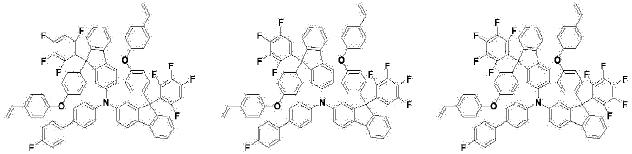

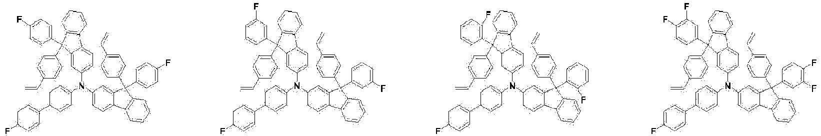

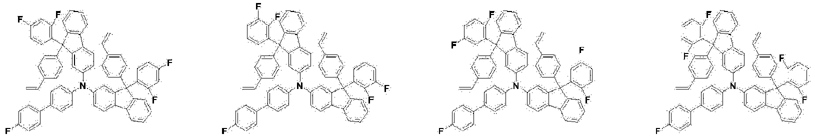

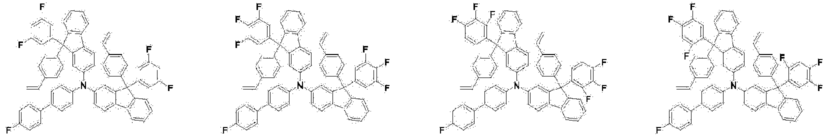

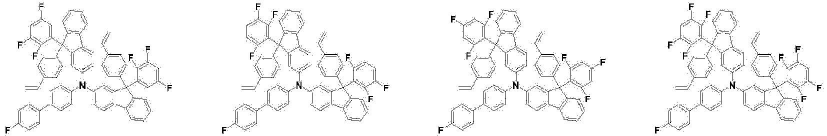

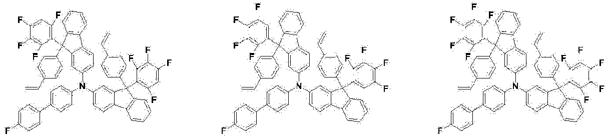

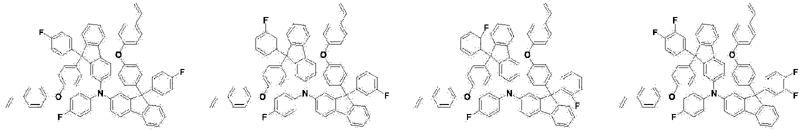

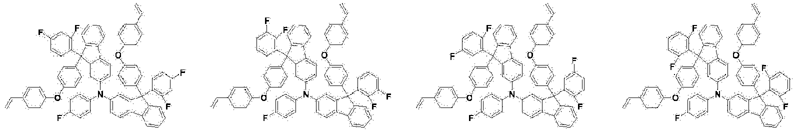

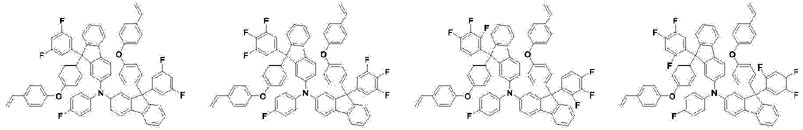

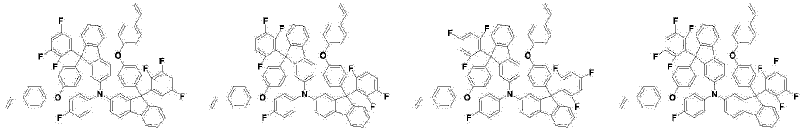

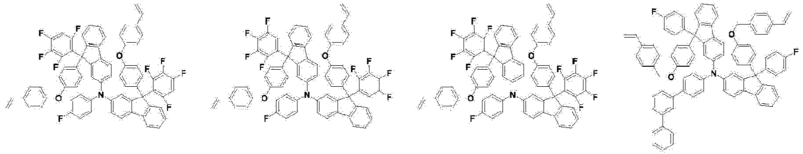

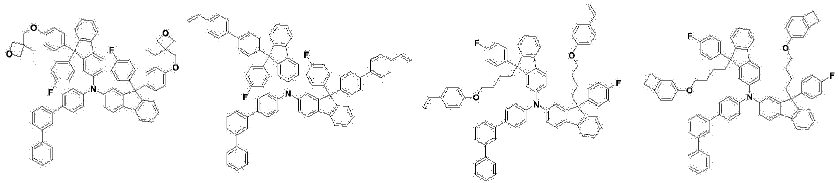

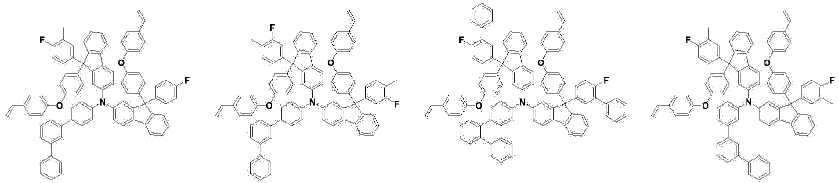

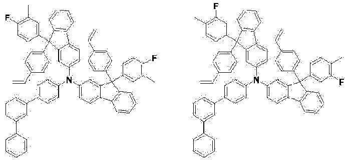

The compound of Chemical Formula 1 according to one embodiment of the present disclosure may have its core structure prepared as in the following Reaction Formula 1. In addition, substituents may bond thereto using methods known in the art, and types, positions and the number of the substituents may vary depending on techniques known in the art.

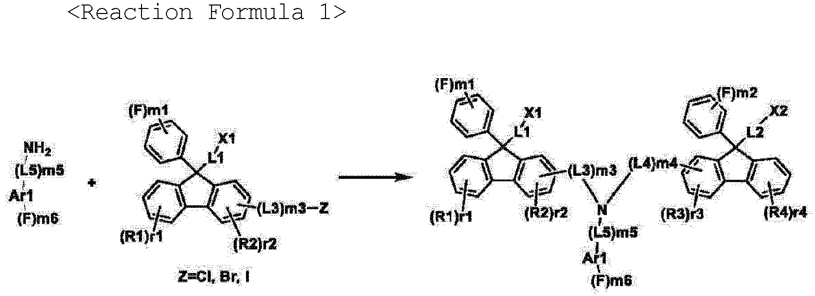

Reaction Formula 1 is an amine substitution reaction, and is preferably conducted under the presence of a palladium catalyst and a base, however, the method is not limited thereto. Reaction groups for the amine substitution reaction of Reaction Formula 1 may be changed using methods known in the art. In Reaction Formula 1, each of other substituents has the same definition as in Chemical Formula 1.

One embodiment of the present disclosure provides a coating composition including the compound of Chemical Formula 1.

According to one embodiment of the present disclosure, the coating composition includes the compound of Chemical Formula 1 and a solvent.

According to one embodiment of the present disclosure, the coating composition may be a liquid phase. The "liquid phase" means being in a liquid state at room temperature and atmospheric pressure.

According to one embodiment of the present disclosure, examples of the solvent may include chlorine-based solvents such as chloroform, methylene chloride, 1,2-dichloroethane, 1,1,2-trichloroethane, chlorobenzene and o-dichlorobenzene; ether-based solvents such as tetrahydrofuran and dioxane; aromatic hydrocarbon-based solvents such as toluene, xylene, trimethylbenzene and mesitylene; aliphatic hydrocarbon-based solvents such as cyclohexane, methylcyclohexane, n-pentane, n-hexane, n-heptane, n-octane, n-nonane and n-decane; ketone-based solvents such as acetone, methyl ethyl ketone, cyclohexanone, isophorone, tetralone, decalone and acetylacetone; ester-based solvents such as ethyl acetate, butyl acetate and ethyl cellosolve acetate; polyalcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin and 1,2-hexanediol, and derivatives thereof; alcohol-based solvents such as methanol, ethanol, propanol, isopropanol and cyclohexanol; sulfoxide-based solvents such as dimethyl sulfoxide; amide-based solvents such as N-methyl-2-pyrrolidone and N,N-dimethylformamide; tetraline, and the like, however, the solvent is not limited thereto as long as it is a solvent capable of dissolving or dispersing the compound of Chemical Formula 1 according to one embodiment of the present disclosure.

According to another embodiment, the solvent may be used either alone as one type, or as a mixture of two or more solvent types.

According to one embodiment of the present disclosure, the solvent may be included in 50% by weight to 99.9% by weight based on the coating composition. Preferably, the solvent may be included in 80% by weight to 99% by weight based on the coating composition, however, the content is not limited thereto.

According to one embodiment of the present disclosure, the coating composition does not further include a p-doping material.

According to one embodiment of the present disclosure, the coating composition further includes a p-doping material.

In the present disclosure, the p-doping material means a material allowing a host material to have p-semiconductor properties. The p-semiconductor properties mean properties receiving or transferring holes at a HOMO (highest occupied molecular orbital) energy level, that is, properties of a material having high hole conductivity.

According to one embodiment of the present disclosure, the p-doping material includes F₄TCNQ; or a boron anion.

According to one embodiment of the present disclosure, the p-doping material includes F₄TCNQ; or a boron anion, and the boron anion includes a halogen group.

According to one embodiment of the present disclosure, the p-doping material includes F₄TCNQ; or a boron anion, and the boron anion includes F.

According to one embodiment of the present disclosure, the p-doping material may be F₄TCNQ or an aryl(fluoro)borate (Farylborate)-based compound like the following Chemical Formulae 4-1 to 4-3, but is not limited thereto.

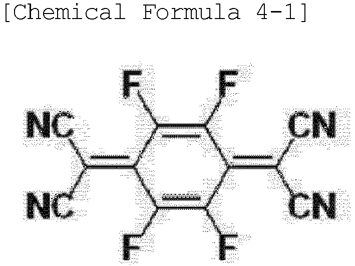

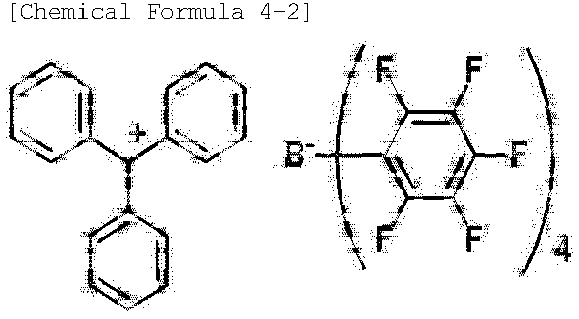

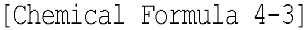

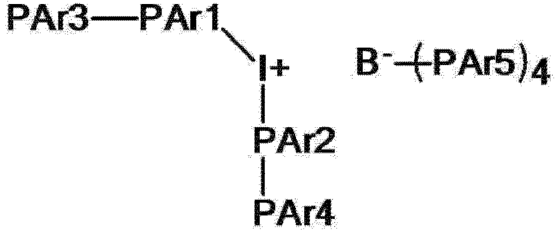

In Chemical Formula 4-3,
PAr1 and PAr2 are the same as or different from each other, and each independently a substituted or unsubstituted aryl group,
PAr3 and PAr4 are the same as or different from each other, and each independently a substituted or unsubstituted alkyl group,
PAr5 is a substituted or unsubstituted aryl group, and
the plurality of PAr5s are the same as or different from each other.

According to one embodiment of the present disclosure, PAr5 may be substituted with a fluoro group.

According to one embodiment of the present disclosure, PAr5 may be substituted with one fluoro group. According to another embodiment of the present disclosure, PAr5 may be substituted with two fluoro groups. According to another embodiment of the present disclosure, PAr5 may be substituted with three fluoro groups. According to another embodiment of the present disclosure, PAr5 may be substituted with four fluoro groups. According to another embodiment of the present disclosure, PAr5 may be substituted with five fluoro groups. According to another embodiment of the present disclosure, PAr5 is a substituted or unsubstituted aryl group having seven or more carbon atoms, and PAr5 may be substituted with six or more fluoro groups.

According to one embodiment of the present disclosure, PAr1 and PAr2 are a substituted or unsubstituted phenyl group, but are not limited thereto.

According to one embodiment of the present disclosure, PAr3 is an isopropyl group. However, the isopropyl group is just an example, and PAr3 is not limited thereto.

According to one embodiment of the present disclosure, PAr4 is a methyl group. However, the methyl group is just an example, and PAr4 is not limited thereto.

According to one embodiment of the present disclosure, PAr5 is a phenyl group substituted with five fluoro groups. However, the phenyl group substituted with five fluoro groups is just an example, and PAr5 is not limited thereto.

As a specific example, Chemical Formula 4-3 may be represented by the following Chemical Formula 4-3-1.

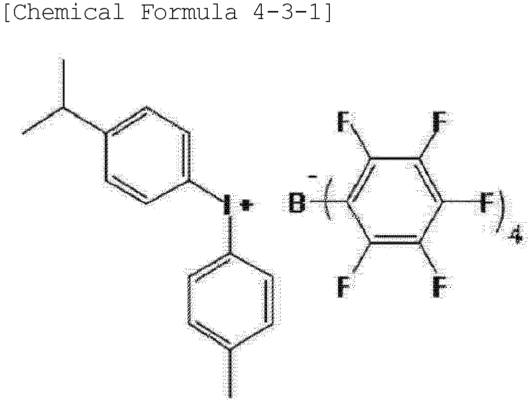

In the present disclosure, the p-doping material suffices as long as it is a material having p-semiconductor properties, and one, two or more types thereof may be used, and types thereof are not limited.

According to one embodiment of the present disclosure, a content of the p-doping material is from 0% by weight to 50% by weight based on the compound of Chemical Formula 1.

According to one embodiment of the present disclosure, the p-doping material is included in 0% by weight to 40% by weight based on a total solid content of the coating composition. According to one embodiment of the present disclosure, the p-doping material is preferably included in 1% by weight to 30% by weight based on a total solid content of the coating composition, and according to another embodiment, the p-doping material is more preferably included in 10% by weight to 30% by weight based on a total solid content of the coating composition.

According to another embodiment, the coating composition may further include a monomer including a photocuring group and/or a thermal curing group; or a monomer including an end group capable of forming a polymer by heat. The monomer including a photocuring group and/or a thermal curing group; or the monomer including an end group capable of forming a polymer by heat as above may be a compound having a molecular weight of 3,000 g/mol or less.

The monomer including a photocuring group and/or a thermal curing group; or the monomer including an end group capable of forming a polymer by heat may mean a monomer having aryl such as phenyl, biphenyl, fluorene or naphthalene; arylamine; or fluorene substituted with a photocuring group and/or a thermal curing group or an end group capable of forming a polymer by heat.

According to another embodiment, the coating composition has viscosity of 2 cP to 15 cP at room temperature (approximately 25°C). When satisfying the above-mentioned viscosity range, a device may be readily manufactured.

In addition, one embodiment of the present disclosure provides an organic light emitting device formed using the coating composition.

According to one embodiment of the present disclosure, the organic light emitting device includes a first electrode; a second electrode; and one or more organic material layers provided between the first electrode and the second electrode, wherein one or more layers of the one or more organic material layers include the coating composition or a cured material thereof, and the cured material of the coating composition is the coating composition being in a cured state by heat treatment or light treatment.

According to one embodiment of the present disclosure, the organic material layer including the coating composition or a cured material thereof is a hole transfer layer.

According to one embodiment of the present disclosure, the organic material layer including the coating composition or a cured material thereof is a hole transfer layer, a hole injection layer or a hole injection and transfer layer.

According to one embodiment of the present disclosure, the organic material layer including the coating composition or a cured material thereof is an electron transfer layer, an electron injection layer or an electron injection and transfer layer.

According to another embodiment, the organic material layer including the coating composition or a cured material thereof is a light emitting layer.

According to another embodiment, the organic material layer including the coating composition or a cured material thereof is a light emitting layer, and the light emitting layer includes the compound of Chemical Formula 1 as a host of the light emitting layer.

According to another embodiment, the organic material layer including the coating composition or a cured material thereof is a light emitting layer, and the light emitting layer includes the compound of Chemical Formula 1 as a dopant of the light emitting layer.

According to one embodiment of the present disclosure, the organic light emitting device further includes one, two or more layers selected from the group consisting of a hole injection layer, a hole transfer layer, an electron transfer layer, an electron injection layer, an electron blocking layer, a hole blocking layer, a hole injection and transfer layer and an electron transfer and injection layer.

According to one embodiment of the present disclosure, the first electrode is an anode, and the second electrode is a cathode.

According to another embodiment, the first electrode is a cathode, and the second electrode is an anode.

According to another embodiment, the organic light emitting device may be an organic light emitting device having a structure in which an anode, one or more organic material layers and a cathode are consecutively laminated on a substrate (normal type).

According to another embodiment, the organic light emitting device may be an organic light emitting device having a structure in a reverse direction in which a cathode, one or more organic material layers and an anode are consecutively laminated on a substrate (inverted type).

The organic material layer of the organic light emitting device of the present disclosure may be formed in a single layer structure, but may be formed in a multilayer structure in which two or more organic material layers are laminated. For example, the organic light emitting device of the present disclosure may have a structure including a hole injection layer, a hole transfer layer, a light emitting layer, an electron transfer layer, an electron injection layer, a hole injection and transfer layer, an electron injection and transfer layer and the like as the organic material layer. However, the structure of the organic light emitting device is not limited thereto, and may include a smaller number of organic layers.

For example, a structure of the organic light emitting device according to one embodiment of the present disclosure is illustrated in FIG. 1.

FIG. 1 illustrates a structure of the organic light emitting device in which a first electrode (201), a hole injection layer (301), a hole transfer layer (401), a light emitting layer (501), an electron injection and transfer layer (601) and a second electrode (701) are consecutively laminated on a substrate (101). Herein, the electron injection and transfer layer means a layer carrying out electron injection and electron transfer at the same time. The hole injection layer (301) and/or the hole transfer layer (401) of FIG. 1 may be formed using the coating composition including the compound of Chemical Formula 1 described above.

FIG. 1 illustrates the organic light emitting device, however, the organic light emitting device is not limited thereto.

When the organic light emitting device includes a plurality of organic material layers, the organic material layers may be formed with materials the same as or different from each other.

The organic light emitting device of the present disclosure may be manufactured using materials and methods known in the art except that one or more layers of the organic material layers are formed using the coating composition including the compound of Chemical Formula 1.

For example, the organic light emitting device of the present disclosure may be manufactured by consecutively laminating an anode, an organic material layer and a cathode on a substrate. Herein, the organic light emitting device may be manufactured by forming an anode on a substrate by depositing a metal, a metal oxide having conductivity, or an alloy thereof using a physical vapor deposition (PVD) method such as sputtering or e-beam evaporation, forming an organic material layer including one or more of a hole injection layer, a hole transfer layer, a light emitting layer, an electron injection layer, an electron transfer layer, a hole injection and transfer layer and an electron injection and transfer layer thereon through a solution process, a deposition process or the like, and then depositing a material usable as a cathode thereon. In addition to such a method, the organic light emitting device may also be manufactured by consecutively depositing a cathode material, an organic material layer and an anode material on a substrate.

In addition, one embodiment of the present disclosure provides a method for manufacturing an organic light emitting device formed using the coating composition.

Specifically, according to one embodiment of the present disclosure, the method includes preparing a first electrode; forming one or more organic material layers on the first electrode; and forming a second electrode on the one or more organic material layers, wherein the forming of one or more organic material layers includes forming one or more organic material layers using the coating composition.

According to one embodiment of the present disclosure, the forming of one or more organic material layers using the coating composition may use a spin coating method, however, the method is not limited thereto.

According to another embodiment, the forming of one or more organic material layers using the coating composition may use a printing method, however, the method is not limited thereto.

In one embodiment of the present disclosure, the printing method may include, for example, inkjet printing, nozzle printing, offset printing, transfer printing, screen printing or the like, but is not limited thereto.

The coating composition according to one embodiment of the present disclosure may be formed using a printing method since a solution process is suited due to structural properties of the coating composition, which is economically effective in terms of time and costs when manufacturing the device.

According to one embodiment of the present disclosure, the forming of one or more organic material layers using the coating composition includes coating the coating composition on the first electrode or the one or more organic material layers; and heat treating or light treating the coated coating composition.

According to one embodiment of the present disclosure, the heat treating may be conducted through heat treatment, and a temperature of the heat treatment in the heat treating may be from 85°C to 250°C, may be from 100°C to 250°C according to another embodiment, and may be from 150°C to 250°C according to still another embodiment. According to preferred one embodiment of the present disclosure, the heat treatment temperature may be 220°C, but is not limited thereto.

According to one embodiment of the present disclosure, a time of the heat treatment in the heat treating may be from 1 minute to 2 hours, may be from 1 minute to 1 hour according to another embodiment, and may be from 30 minutes to 1 hour according to still another embodiment. According to preferred one example of the present disclosure, the heat treatment time may be 30 minutes, but is not limited thereto.

When including the heat treating or light treating in the forming of one or more organic material layers formed using the coating composition, a plurality of the compounds included in the coating composition form crosslinks, and an organic material layer including a thin-filmed structure may be provided. This may prevent the organic material layer formed using the coating composition from being dissolved by a solvent, being morphologically influenced or being decomposed when laminating other layers on the surface of the organic material layer. Accordingly, when the organic material layer formed using the coating composition is formed including the heat treating or light treating, resistance for a solvent increases, and a multilayer may be formed by repeatedly performing solution deposition and crosslinking methods, and lifetime properties of the device may be enhanced by increasing stability.

As the anode material, materials having large work function are normally preferred so that hole injection to an organic material layer is smooth. Specific examples of the anode material usable in the present disclosure include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having small work function are normally preferred so that electron injection to an organic material layer is smooth. Specific examples of the cathode material include metals such as barium, magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

The hole injection layer is a layer that injects holes from an electrode, and the hole injection material is preferably a compound that has an ability to transfer holes and thereby has a hole injection effect in an anode and an excellent hole injection effect for a light emitting layer or a light emitting material, prevents excitons generated in the light emitting layer from moving to an electron injection layer or an electron injection material, and in addition thereto, has an excellent thin film forming ability. The highest occupied molecular orbital (HOMO) of the hole injection material is preferably in between the work function of an anode material and the HOMO of surrounding organic material layers. Specific examples of the hole injection material include the compound of Chemical Formula 1 described above, metal porphyrins, oligothiophene, arylamine-based organic materials, hexanitrile hexaazatriphenylene-based organic materials, quinacridone-based organic materials, perylene-based organic materials, anthraquinone, and polyaniline- and polythiophene-based conductive polymers, and the like, but are not limited thereto.

The hole transfer layer is a layer that receives holes from a hole injection layer and transfers the holes to a light emitting layer, and as the hole transfer material, materials capable of receiving holes from an anode or a hole injection layer, moving the holes to a light emitting layer, and having high mobility for the holes are suited. Specific examples thereof include arylamine-based organic materials, conductive polymers, block copolymers having conjugated parts and nonconjugated parts together, and the like, but are not limited thereto.

The hole injection and transfer layer may include the materials of the hole transfer layer and the hole injection layer described above.

The light emitting material is a material capable of emitting light in a visible light region by receiving holes and electrons from a hole transfer layer and an electron transfer layer, respectively, and binding the holes and the electrons, and is preferably a material having favorable quantum efficiency for fluorescence or phosphorescence. Specific examples thereof include 8-hydroxyquinoline aluminum complexes (Alq₃); carbazole-based compounds; dimerized styryl compounds; BAlq; 10-hydroxybenzoquinoline-metal compounds; benzoxazole-, benzothiazole- and benzimidazole-based compounds; poly(p-phenylenevinylene) (PPV)-based polymers; spiro compounds; polyfluorene, rubrene, or the like, but are not limited thereto.

The light emitting layer may include a host material and a dopant material. The host material includes fused aromatic ring derivatives, heteroring-containing compounds or the like. Specifically, the fused aromatic ring derivative includes anthracene derivatives, pyrene derivatives, naphthalene derivatives, pentacene derivatives, phenanthrene compounds, fluoranthene compounds and the like, and the heteroring-containing compound includes carbazole derivatives, dibenzofuran derivatives, ladder-type furan compounds, pyrimidine derivatives and the like, however, the material is not limited thereto.

The dopant material includes aromatic amine derivatives, styrylamine compounds, boron complexes, fluoranthene compounds, metal complexes and the like. Specifically, the aromatic amine derivative is a fused aromatic ring derivative having a substituted or unsubstituted arylamino group and includes arylamino group-including pyrene, anthracene, chrysene, peryflanthene and the like, and the styrylamine compound is a compound in which substituted or unsubstituted arylamine is substituted with at least one arylvinyl group, and one, two or more substituents selected from the group consisting of an aryl group, a silyl group, an alkyl group, a cycloalkyl group and an arylamino group are substituted or unsubstituted. Specifically, styrylamine, styryldiamine, styryltriamine, styryltetramine or the like is included, however, the styrylamine compound is not limited thereto. In addition, the metal complex includes iridium complexes, platinum complexes or the like, but is not limited thereto.

The electron transfer layer is a layer that receives electrons from an electron injection layer and transfers the electrons to a light emitting layer, and as the electron transfer material, materials capable of favorably receiving electrons from a cathode, moving the electrons to a light emitting layer, and having high mobility for the electrons are suited. Specific examples thereof include Al complexes of 8-hydroxyquinoline; complexes including Alq₃; organic radical compounds; hydroxyflavon-metal complexes, or the like, but are not limited thereto. The electron transfer layer may be used together with any desired cathode material as used in the art. Particularly, examples of the suitable cathode material include common materials that have small work function, and in which an aluminum layer or a silver layer follows. Specifically, the cathode material includes cesium, barium, calcium, ytterbium and samarium, and in each case, an aluminum layer or a silver layer follows.

The electron injection layer is a layer that injects electrons from an electrode, and as the electron injection material, compounds that has an ability to transfer electrons, has an electron injection effect from a cathode, has an excellent electron injection effect for a light emitting layer or a light emitting material, prevents excitons generated in the light emitting layer from moving to a hole injection layer, and in addition thereto, has an excellent thin film forming ability are preferred. Specific examples thereof include fluorenone, anthraquinodimethane, diphenoquinone, thiopyran dioxide, oxazole, oxadiazole, triazole, imidazole, perylene tetracarboxylic acid, fluorenylidene methane, anthrone or the like, and derivatives thereof, metal complex compounds, nitrogen-containing 5-membered ring derivatives, and the like, but are not limited there.

The electron injection and transfer layer may include the materials of the electron transfer layer and the electron injection layer described above.

The metal complex compound includes 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato) (o-cresolato)gallium, bis(2-methyl-8-quinolinato)(1-naphtholato)aluminum, bis(2-methyl-8-quinolinato)(2-naphtholato)gallium and the like, but is not limited thereto.

The hole blocking layer is a layer blocking or suppressing holes from reaching a cathode, and may be generally formed under the same condition as the hole injection layer. Specific examples thereof may include oxadiazole derivatives or triazole derivatives, phenanthroline derivatives, BCP, aluminum complexes and the like, but are not limited thereto.

The electron blocking layer is a layer blocking or suppressing electrons from reaching an anode, and materials known in the art may be used.

The organic light emitting device according to the present disclosure may be a top-emission type, a bottom-emission type or a dual-emission type depending on the materials used.

Unless defined otherwise in the present specification, all technical and scientific terms used in the present disclosure have the same meanings as terms commonly understood by those skilled in the art. Although methods and materials similar or equivalent to those described in the present disclosure may be used in implementing or experimenting embodiments of the present disclosure, suitable methods and materials are described later. All publications, patent applications, patents and other reference documents mentioned in the present disclosure are incorporated by reference in the present disclosure as a whole, and when conflicting, the present disclosure including definitions has priority unless specific passage is mentioned. Furthermore, materials, methods and examples are for illustrative purposes only, and not to limit the present disclosure.

Hereinafter, the present disclosure will be described in detail with reference to examples in order to specifically describe the present disclosure. However, the examples according to the present disclosure may be modified to various different forms, and the scope of the present disclosure is not to be construed as being limited to the examples described below. Examples of the present disclosure are provided in order to more fully describe the present disclosure to those having average knowledge in the art.

### <Preparation Example>

### Preparation Example 1. Preparation of Compound 1

### 1) Synthesis of Intermediate 1-1

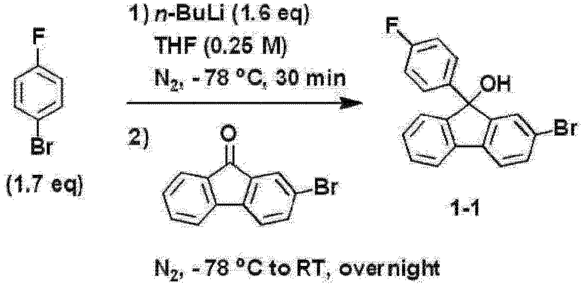

1-Bromo-4-fluorobenzene (27.9 mL, 255 mmol) was introduced to tetrahydrofuran (THF) (500 mL). The flask was substituted with nitrogen and cooled to -78°C. n-Butyllithium (n-BuLi) (2.5 M Hex) (96 mL, 240 mmol) was introduced to a dropping funnel, and then slowly introduced to the reaction mixture. The result was stirred for 30 minutes at -78°C. 2-Bromofluorenone (38.9 g, 150 mmol) was introduced thereto. The result was stirred overnight while slowly raising the temperature to room temperature. The reaction was terminated by introducing distilled water thereto, and the result was extracted with ethyl acetate and water. The organic layer was collected, dried using MgSO₄, and filtered. The filtrate was dried using a vacuum rotary concentrator to remove the organic solvent, and used for the next reaction.

### 2) Synthesis of Intermediate 1-2

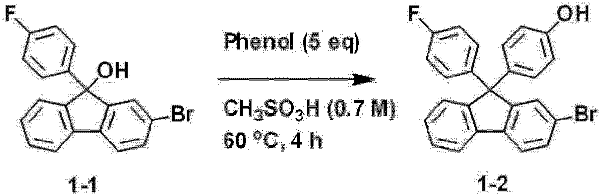

Intermediate 1-1 (53 g, 150 mmol) and phenol (70.6 g, 750 mmol) were introduced to a round bottom flask (RBF) . CH₃SO₃H (214 mL) was introduced thereto, and the mixture was stirred for 4 hours at 60°C. Ice water was introduced thereto, and the result was extracted with ethyl acetate and water. The organic layer was collected, dried using MgSO₄, and filtered. The filtrate was dried using a vacuum rotary concentrator to remove the organic solvent. The result was column purified, and then crystallized under a condition of dichloromethane (DCM)/heptane to secure Intermediate 1-2 (40.6 g).

### 3) Synthesis of Intermediate 1-3

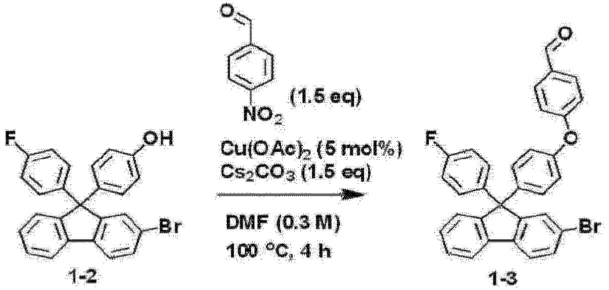

Intermediate 1-2 (40 g, 92.7 mmol), 4-nitrobenzaldehyde (21.2 g, 139 mmol), Cu(OAc)₂ (842 mg, 4.64 mmol) and Cs₂CO₃ (45.3 g, 139 mmol) were introduced to a round bottom flask (RBF) . Dimethylformamide (DMF) (310 mL) was introduced thereto, and the mixture was stirred for 4 hours at 100°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/heptane to secure Intermediate 1-3 (38.7 g).

### 4) Synthesis of Intermediate 1-4

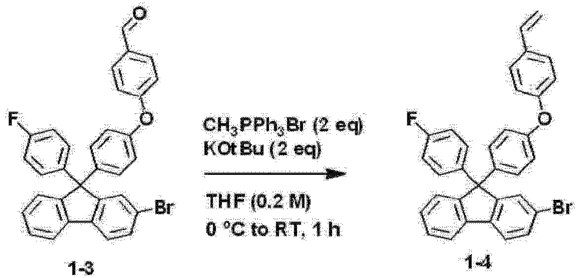

CH₃PPh₃Br (51.6 g, 144.6 mmol), potassium tert-butoxide (KOtBu) (16.2 g, 144.6 mmol) and tetrahydrofuran (THF) (217 mL) were introduced to a round bottom flask (RBF), and cooled to 0°C. A solution obtained by dissolving Intermediate 1-3 (38.7 g, 72.3 mmol) in tetrahydrofuran (THF) (144 mL) was introduced to the reaction mixture. The result was stirred for 1 hour while raising the temperature to room temperature. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Intermediate 1-4 (33.7 g).

### 5) Synthesis of Compound 1

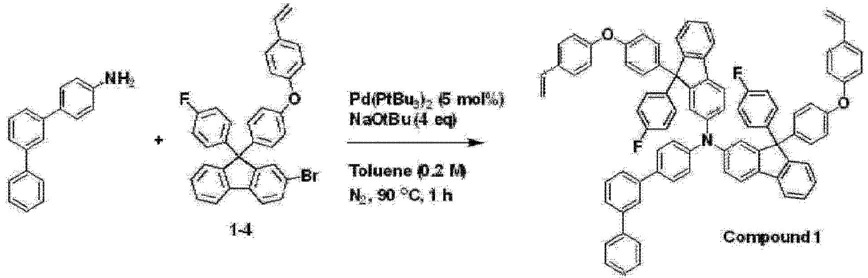

[1,1':3',1"-Terphenyl]-4-amine (687 mg, 2.8 mmol), Intermediate 1-4 (3.1 g, 5.74 mmol), Pd(PtBu₃)₂ (72 mg, 0.14 mmol) and sodium tert-butoxide (NaOtBu) (1.08 g, 11.2 mmol) were introduced to a round bottom flask (RBF). The flask was substituted with nitrogen, toluene (14 mL) was introduced thereto, and the mixture was stirred for 1 hour at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Compound 1 (2.8 g). Synthesis of the compound was identified through LC-MS and NMR. MS: [M+H]⁺=1150
NMR measurement values of Compound 1: ¹H NMR (500 MHz, DMSO-d6) δ 7.84-7.81 (m, 5H), 7.71 (d, 2H), 7.61-7.35 (m, 16H), 7.28 (t, 2H), 7.12-7.00 (m, 18H), 6.92 (d, 4H), 6.86 (t, 4H), 6.64 (m, 2H), 5.67 (dd, 2H), 5.17 (dd, 2H)

### Preparation Example 2. Preparation of Compound 2

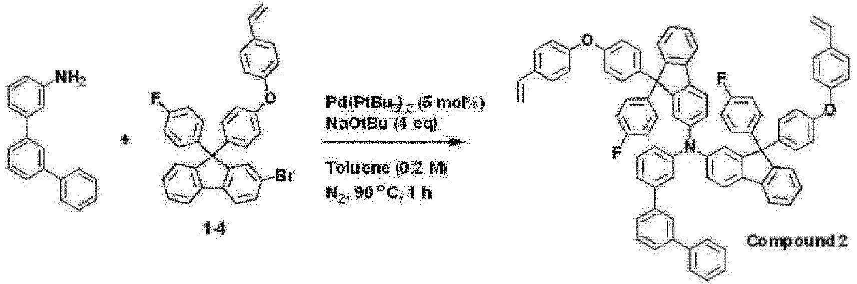

[1,1':3',1"-Terphenyl]-3-amine (687 mg, 2.8 mmol), Intermediate 1-4 (3.1 g, 5.74 mmol), Pd(PtBu₃)₂ (72 mg, 0.14 mmol) and sodium tert-butoxide (NaOtBu) (1.08 g, 11.2 mmol) were introduced to a round bottom flask (RBF). The flask was substituted with nitrogen, toluene (14 mL) was introduced thereto, and the mixture was stirred for 1 hour at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Compound 2 (2.6 g). Synthesis of the compound was identified through LC-MS and NMR. MS: [M+H]⁺=1150
NMR measurement values of Compound 2: ¹H NMR (500 MHz, DMSO-d6) δ 7.84-7.81 (m, 5H), 7.71 (d, 2H), 7.62-7.35 (m, 17H), 7.28 (t, 2H), 7.13-7.00 (m, 17H), 6.92 (d, 4H), 6.86 (t, 4H), 6.64 (m, 2H), 5.67 (dd, 2H), 5.17 (dd, 2H)

### Preparation Example 3. Preparation of Compound 3

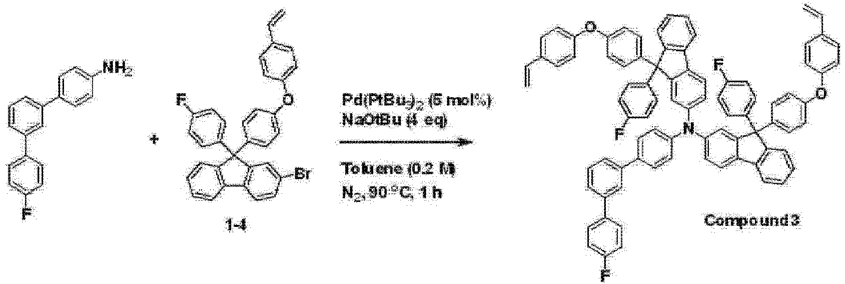

4"-Fluoro-[1,1':3',1"-terphenyl]-4-amine (737 mg, 2.8 mmol), Intermediate 1-4 (3.1 g, 5.74 mmol), Pd(PtBu₃)₂ (72 mg, 0.14 mmol) and sodium tert-butoxide (NaOtBu) (1.08 g, 11.2 mmol) were introduced to a round bottom flask (RBF). The flask was substituted with nitrogen, toluene (14 mL) was introduced thereto, and the mixture was stirred for 1 hour at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Compound 3 (2.9 g). Synthesis of the compound was identified through LC-MS and NMR. MS: [M+H]⁺=1168
NMR measurement values of Compound 3: ¹H NMR (500 MHz, DMSO-d6) δ 7.84-7.77 (m, 7H), 7.61-7.35 (m, 15H), 7.28 (t, 2H), 7.12-7.00 (m, 18H), 6.92 (d, 4H), 6.86 (t, 4H), 6.64 (m, 2H), 5.67 (dd, 2H), 5.17 (dd, 2H)

### Preparation Example 4. Preparation of Compound 4

### 1) Preparation of Intermediate 4-1

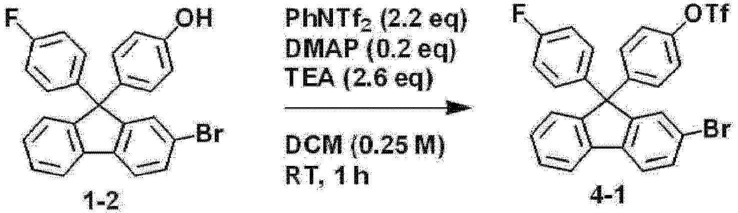

Intermediate 1-2 (45 g, 104 mmol), phenyl triflimide (81.8 g, 229 mmol) and 4-dimethylaminopyridine (DMAP) (2.54 g, 21 mmol) were introduced to a round bottom flask. Dichloromethane (416 mL) and triethylamine (37.7 mL, 270 mmol) were introduced thereto, and the mixture was stirred for 1 hour at room temperature. The result was extracted with dichloromethane and a 3% aqueous HCl solution, and the organic layer was collected, dried using MgSO₄ and then filtered. The filtrate was dried using a vacuum rotary concentrator to remove the organic solvent. The result was crystallized under a condition of dichloromethane (DCM)/heptane to prepare Intermediate 4-1 (52.5 g).

### 2) Preparation of Intermediate 4-2

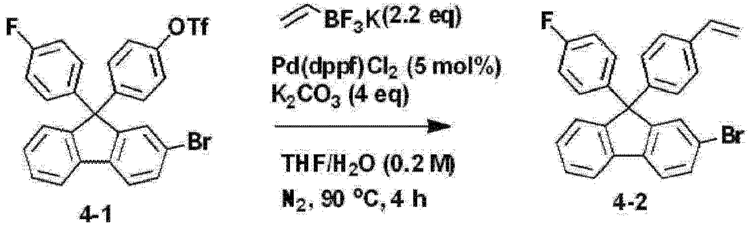

Intermediate 4-1 (52.2 g, 92.7 mmol), potassium vinyltrifluoroborate (27.3 g, 204 mmol), Pd(dppf)Cl₂ (3.4 g, 4.6 mmol) and K₂CO₃ (51.2 g, 971 mmol) were introduced to a round bottom flask. The flask was substituted with nitrogen, tetrahydrofuran (THF) (371 mL) and H₂O (93 mL) were introduced thereto, and the mixture was stirred for 4 hours at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to prepare Intermediate 4-2 (34 g).

### 3) Preparation of Compound 4

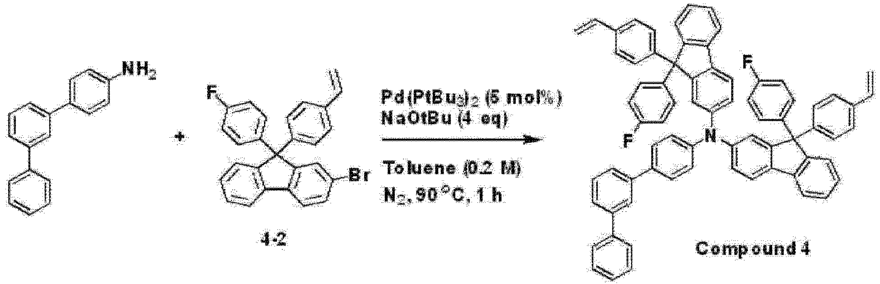

[1,1':3',1"-Terphenyl]-4-amine (687 mg, 2.8 mmol), Intermediate 4-2 (2.53 g, 5.74 mmol), Pd(PtBu₃)₂ (72 mg, 0.14 mmol) and sodium tert-butoxide (NaOtBu) (1.08 g, 11.2 mmol) were introduced to a round bottom flask (RBF). The flask was substituted with nitrogen, toluene (14 mL) was introduced thereto, and the mixture was stirred for 1 hour at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Compound 4 (2.2 g). Synthesis of the compound was identified through LC-MS and NMR. MS: [M+H]⁺=966
NMR measurement values of Compound 4: ¹H NMR (500 MHz, DMSO-d6) δ 7.85-7.82 (m, 5H), 7.70 (d, 2H), 7.61-7.35 (m, 16H), 7.28 (t, 2H), 7.12-7.00 (m, 14H), 6.84 (t, 4H), 6.62 (dd, 2H), 5.63 (dd, 2H), 5.14 (dd, 2H)

### Preparation Example 5. Preparation of Compound 5

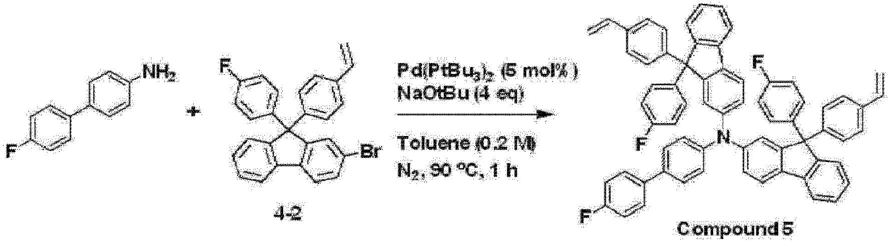

4'-Fluoro-[1,1'-biphenyl]-4-amine (524 mg, 2.8 mmol), Intermediate 4-2 (2.53 g, 5.74 mmol), Pd(PtBu₃)₂ (72 mg, 0.14 mmol) and sodium tert-butoxide (NaOtBu) (1.08 g, 11.2 mmol) were introduced to a round bottom flask (RBF). The flask was substituted with nitrogen, toluene (14 mL) was introduced thereto, and the mixture was stirred for 1 hour at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Compound 5 (2.1 g). Synthesis of the compound was identified through LC-MS and NMR. MS: [M+H]⁺=908
NMR measurement values of Compound 5: ¹H NMR (500 MHz, DMSO-d6) δ 7.85-7.82 (m, 5H), 7.78 (d, 2H), 7.61-7.35 (m, 11H), 7.28 (t, 2H), 7.12-7.00 (m, 14H), 6.84 (t, 4H), 6.62 (dd, 2H), 5.63 (dd, 2H), 5.14 (dd, 2H)

### Preparation Example 6. Preparation of Compound 6

### 1) Preparation of Intermediate 6-1

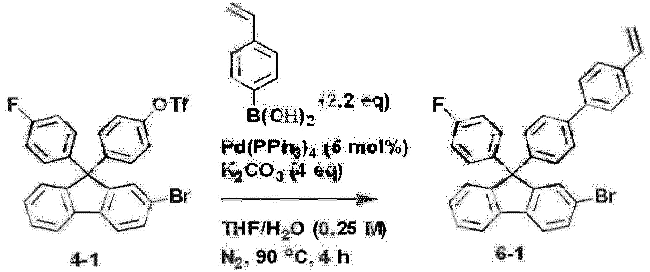

Intermediate 4-1(11.3 g, 20 mmol), 4-vinylphenylboronic acid (6.51 g, 44 mmol), Pd(PPh₃)₄ (1.16 g, 1 mmol) and K₂CO₃ (11 g, 80 mmol) were introduced to a round bottom flask. After that, the flask was substituted with nitrogen, and tetrahydrofuran (THF) (64 mL) and H₂O (16 mL) were introduced thereto. The mixture was stirred for 4 hours at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to prepare Intermediate 6-1 (7 g).

### 2) Preparation of Compound 6

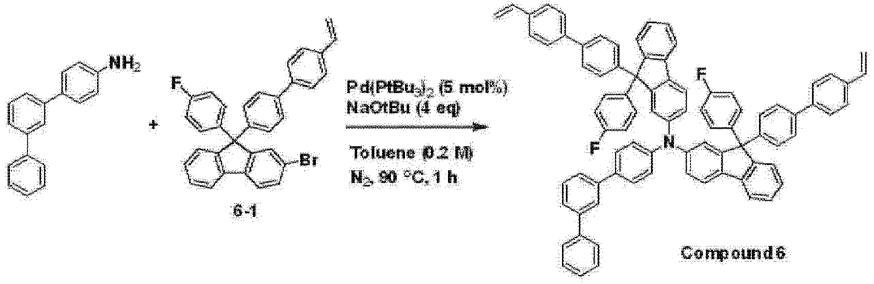

[1,1':3',1"-Terphenyl]-4-amine) (687 mg, 2.8 mmol), Intermediate 6-1 (2.97 g, 5.74 mmol), Pd(PtBu₃)₂ (72 mg, 0.14 mmol) and sodium tert-butoxide (NaOtBu) (1.08 g, 11.2 mmol) were introduced to a round bottom flask (RBF). The flask was substituted with nitrogen, toluene (14 mL) was introduced thereto, and the mixture was stirred for 1 hour at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Compound 6 (2.6 g). Synthesis of the compound was identified through LC-MS and NMR. MS: [M+H]⁺=1118
NMR measurement values of Compound 6: ¹H NMR (500 MHz, DMSO-d6) δ 7.84-7.81 (m, 5H), 7.71 (d, 2H), 7.61-7.35 (m, 16H), 7.28 (t, 2H), 7.12-6.90 (m, 18H), 6.87 (d, 4H), 6.81 (t, 4H), 6.62 (dd, 2H), 5.63 (dd, 2H), 5.14 (dd, 2H)

### Preparation Example 7. Preparation of Compound 7

### 1) Synthesis of Intermediate 7-1

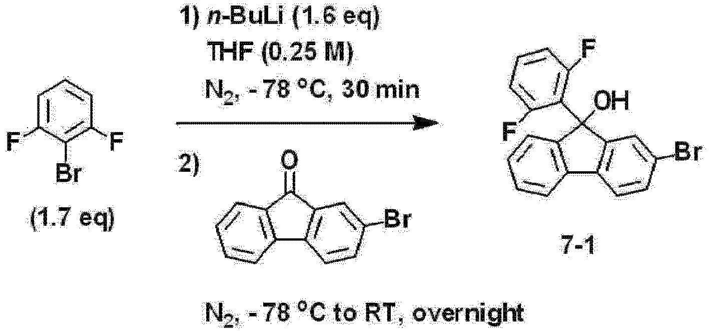

1-Bromo-2,6-difluorobenzene (29.4 mL, 255 mmol) was introduced to tetrahydrofuran (THF) (500 mL). The flask was substituted with nitrogen, and cooled to -78°C. n-Butyllithium (n-BuLi) (2.5 M Hex) (96 mL, 240 mmol) was introduced to a dropping funnel, and then slowly introduced to the reaction mixture. The result was stirred for 30 minutes at -78°C. 2-Bromofluorenone (38.9 g, 150 mmol) was introduced thereto, and the result was stirred overnight while slowly raising the temperature to room temperature. The reaction was terminated by introducing distilled water thereto, and the result was extracted with ethyl acetate and water. The organic layer was collected, dried using MgSO₄, and filtered. The filtrate was dried using a vacuum rotary concentrator to remove the organic solvent, and used for the next reaction.

### 2) Synthesis of Intermediate 7-2

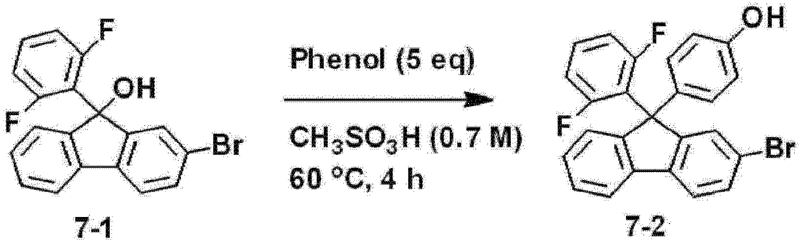

Intermediate 7-1 (56 g, 150 mmol) and phenol (70.6 g, 750 mmol) were introduced to a round bottom flask (RBF) . CH₃SO₃H (214 mL) was introduced thereto, and the mixture was stirred for 4 hours at 60°C. Ice water was introduced thereto, and the result was extracted with ethyl acetate and water. The organic layer was collected, dried using MgSO₄, and filtered. The filtrate was dried using a vacuum rotary concentrator to remove the organic solvent. The result was column purified, and then crystallized under a condition of dichloromethane (DCM)/heptane to secure Intermediate 7-2 (45.2 g).

### 3) Synthesis of Intermediate 7-3

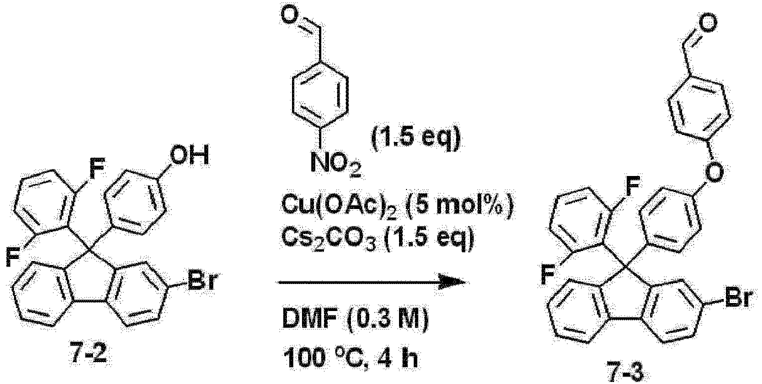

Intermediate 7-2 (45.2 g, 100 mmol), 4-nitrobenzaldehyde (22.8 g, 150 mmol), copper acetate (Cu(OAc)₂) (908 mg, 5 mmol) and Cs₂CO₃ (48.9 g, 150 mmol) were introduced to a round bottom flask (RBF) . Dimethylformamide (DMF) (333 mL) was introduced thereto, and the mixture was stirred for 4 hours at 100°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/heptane to secure Intermediate 7-3 (39.8 g).

### 4) Synthesis of Intermediate 7-4

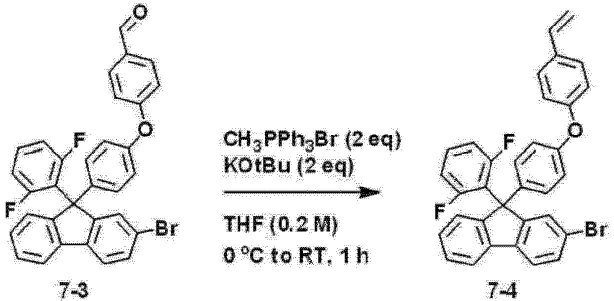

CH₃PPh₃Br (51.4 g, 144 mmol), potassium tert-butoxide (KOtBu) (16.2 g, 144 mmol) and THF (216 mL) were introduced to RBF, and cooled to 0°C. A solution obtained by dissolving Intermediate 7-3 (39.8 g, 72 mmol) in tetrahydrofuran (THF) (144 mL) was introduced to the reaction mixture, and the result was stirred for 1 hour while raising the temperature to room temperature. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Intermediate 7-4 (34.8 g).

### 5) Synthesis of Compound 7

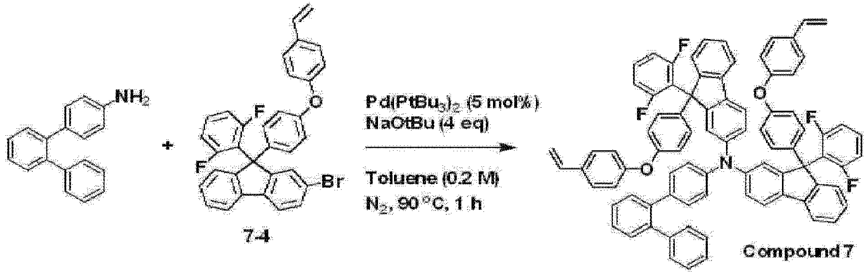

[1,1':2',1"-Terphenyl]-4-amine (687 mg, 2.8 mmol), Intermediate 7-4 (3.2 g, 5.74 mmol), Pd(PtBu₃)₂ (72 mg, 0.14 mmol) and sodium tert-butoxide (NaOtBu) (1.08 g, 11.2 mmol) were introduced to a round bottom flask (RBF). The flask was substituted with nitrogen, toluene (14 mL) was introduced thereto, and the mixture was stirred for 1 hour at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Compound 7 (2.8 g). Synthesis of the compound was identified through LC-MS and NMR. MS: [M+H]⁺=1186
NMR measurement values of Compound 7: ¹H NMR (500 MHz, DMSO-d6) δ 7.84-7.81 (m, 5H), 7.71 (d, 2H), 7.61-7.35 (m, 16H), 7.28 (t, 2H), 7.20-7.08 (m, 16H), 6.92 (d, 4H), 6.86 (t, 4H), 6.64 (m, 2H), 5.67 (dd, 2H), 5.17 (dd, 2H)

### Preparation Example 8. Preparation of Compound 8

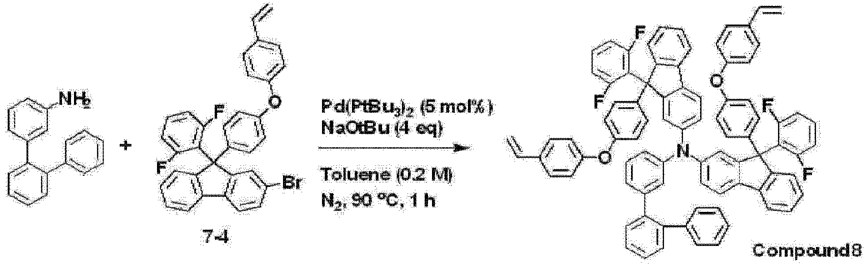

[1,1':2',1"-Terphenyl]-3-amine (687 mg, 2.8 mmol), Intermediate 7-4 (3.2 g, 5.74 mmol), Pd(PtBu₃)₂ (72 mg, 0.14 mmol) and sodium tert-butoxide (NaOtBu) (1.08 g, 11.2 mmol) were introduced to a round bottom flask (RBF). The flask was substituted with nitrogen, toluene (14 mL) was introduced thereto, and the mixture was stirred for 1 hour at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Compound 8 (2.9 g). Synthesis of the compound was identified through LC-MS and NMR. MS: [M+H]⁺=1186
NMR measurement values of Compound 8: ¹H NMR (500 MHz, DMSO-d6) δ 7.85-7.81 (m, 5H), 7.72 (d, 2H), 7.61-7.35 (m, 16H), 7.28 (t, 2H), 7.20-7.08 (m, 16H), 6.92 (d, 4H), 6.86 (t, 4H), 6.64 (m, 2H), 5.67 (dd, 2H), 5.17 (dd, 2H)

### Preparation Example 9. Preparation of Compound 9

### 1) Synthesis of Intermediate 9-1

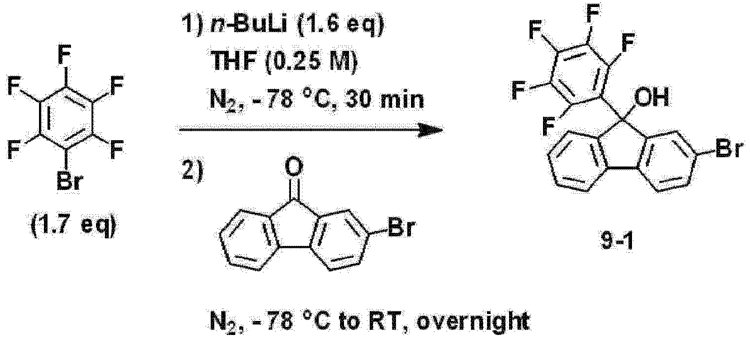

1-Bromo-2,3,4,5-tetrafluorobenzene (31.8 mL, 255 mmol) was introduced to tetrahydrofuran (THF) (500 mL), and the flask was substituted with nitrogen and cooled to -78°C. n-Butyllithium (n-BuLi) (2.5 M Hex) (96 mL, 240 mmol) was introduced to a dropping funnel, then slowly introduced to the reaction mixture, and the result was stirred for 30 minutes at -78°C. 2-Bromofluorenone (38.9 g, 150 mmol) was introduced thereto. The result was stirred overnight while slowly raising the temperature to room temperature. The reaction was terminated by introducing distilled water thereto, and the result was extracted with ethyl acetate and water. The organic layer was collected, dried using MgSO₄, and filtered. The filtrate was dried using a vacuum rotary concentrator to remove the organic solvent, and used for the next reaction.

### 2) Synthesis of Intermediate 9-2

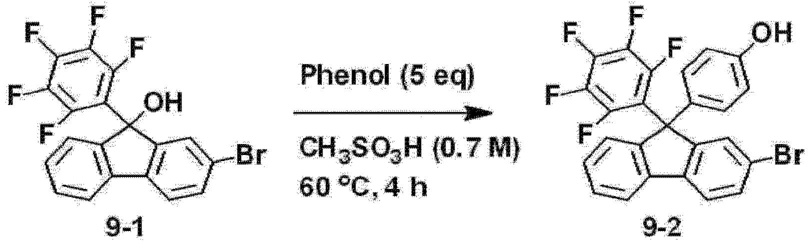

Intermediate 9-1 (64.1 g, 150 mmol) and phenol (70.6 g, 750 mmol) were introduced to a round bottom flask (RBF). CH₃SO₃H (214 mL) was introduced thereto, and the mixture was stirred for 4 hours at 60°C. Ice water was introduced thereto, and the result was extracted with ethyl acetate and water. The organic layer was collected, dried using MgSO₄, and filtered. The filtrate was dried using a vacuum rotary concentrator to remove the organic solvent. The result was column purified, and then crystallized under a condition of dichloromethane (DCM)/heptane to secure Intermediate 9-2 (52.8 g).

### 3) Synthesis of Intermediate 9-3

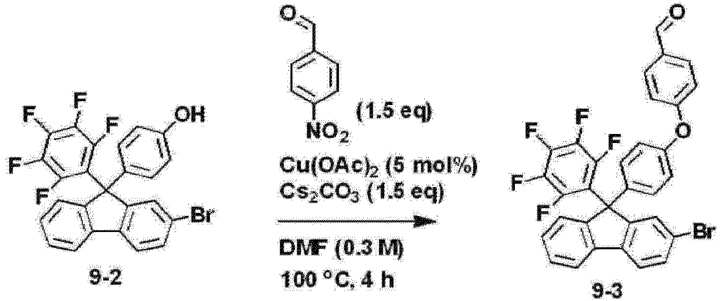

Intermediate 9-2 (50.3 g, 100 mmol), 4-nitrobenzaldehyde (22.8 g, 150 mmol), Cu(OAc)₂ (908 mg, 5 mmol) and Cs₂CO₃ (48.9 g, 150 mmol) were introduced to a round bottom flask (RBF). Dimethylformamide (DMF) (333 mL) was introduced thereto, and the mixture was stirred for 4 hours at 100°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/heptane to secure Intermediate 9-3 (48.6 g).

### 4) Synthesis of Intermediate 9-4

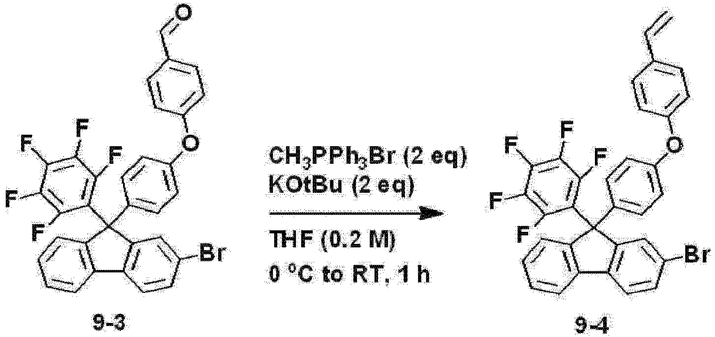

CH₃PPh₃Br (51.4 g, 144 mmol), potassium tert-butoxide (KOtBu) (16.2 g, 144 mmol) and tetrahydrofuran (THF) (216 mL) were introduced to a round bottom flask (RBF), and cooled to 0°C. A solution obtained by dissolving Intermediate 9-3 (43.7 g, 72 mmol) in tetrahydrofuran (THF) (144 mL) was introduced to the reaction mixture, and the result was stirred for 1 hour while raising the temperature to room temperature. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Intermediate 9-4 (31 g).

### 5) Synthesis of Compound 9

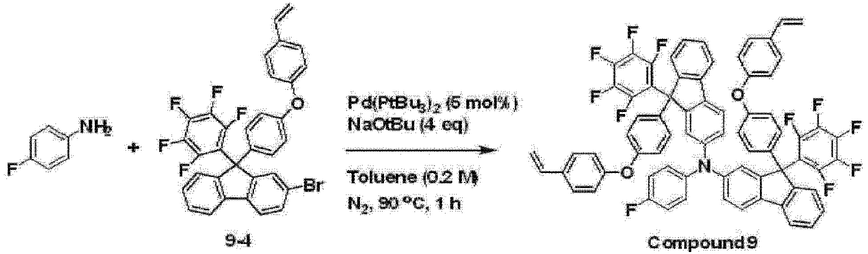

4-Fluoroaniline (0.26 mL, 2.8 mmol), Intermediate 9-4 (3.5 g, 5.74 mmol), Pd(PtBu₃)₂ (72 mg, 0.14 mmol) and sodium tert-butoxide (NaOtBu) (1.08 g, 11.2 mmol) were introduced to a round bottom flask (RBF). The flask was substituted with nitrogen, toluene (14 mL) was introduced thereto, and the mixture was stirred for 1 hour at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Compound 9 (2.4 g). Synthesis of the compound was identified through LC-MS and NMR. MS: [M+H]⁺=1160
NMR measurement values of Compound 9: ¹H NMR (500 MHz, DMSO-d6) δ 7.81 (m, 2H), 7.58-7.35 (m, 11H), 7.28 (t, 2H), 7.15-7.09 (m, 11H), 6.92 (d, 4H), 6.86 (t, 4H), 6.64 (m, 2H), 5.67 (dd, 2H), 5.17 (dd, 2H)

### Preparation Example 10. Preparation of Compound 10

### 1) Synthesis of Intermediate 10-1

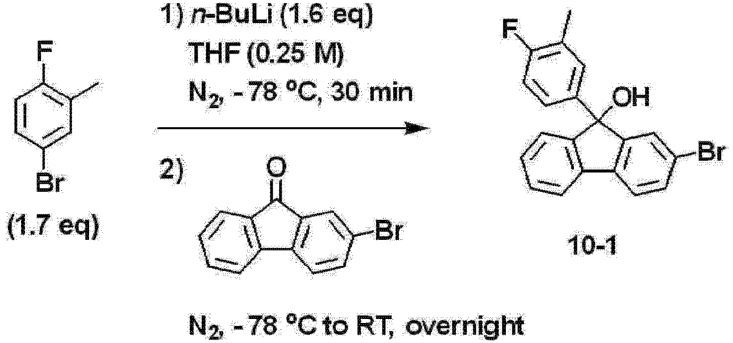

5-Bromo-2-fluorobenzene (31.7 mL, 255 mmol) was introduced to tetrahydrofuran (THF) (500 mL), and the flask was substituted with nitrogen and cooled to -78°C. n-Butyllithium (n-BuLi) (2.5 M Hex) (96 mL, 240 mmol) was introduced to a dropping funnel, then slowly introduced to the reaction mixture, and the result was stirred for 30 minutes at -78°C. 2-Bromofluorenone (38.9 g, 150 mmol) was introduced thereto. The result was stirred overnight while slowly raising the temperature to room temperature. The reaction was terminated by introducing distilled water thereto, and the result was extracted with ethyl acetate and water. The organic layer was collected, dried using MgSO₄, and filtered. The filtrate was dried using a vacuum rotary concentrator to remove the organic solvent, and used for the next reaction.

### 2) Synthesis of Intermediate 10-2

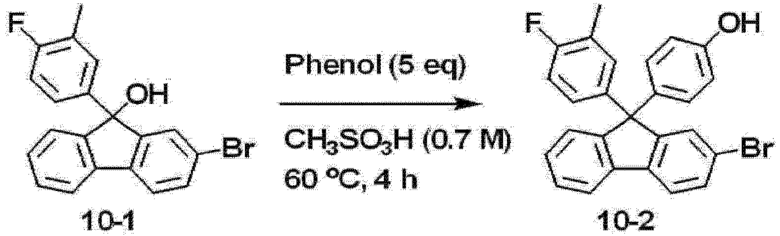

Intermediate 10-1 (55.4 g, 150 mmol) and phenol (70.6 g, 750 mmol) were introduced to a round bottom flask (RBF). CH₃SO₃H (214 mL) was introduced thereto, and the mixture was stirred for 4 hours at 60°C. Ice water was introduced thereto, and the result was extracted with ethyl acetate and water. The organic layer was collected, dried using MgSO₄, and filtered. The filtrate was dried using a vacuum rotary concentrator to remove the organic solvent. The result was column purified, and then crystallized under a condition of dichloromethane (DCM)/heptane to secure Intermediate 10-2 (44.8 g).

### 3) Synthesis of Intermediate 10-3

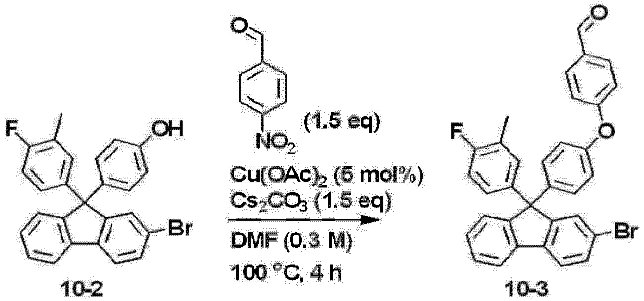

Intermediate 10-2 (44.5 g, 100 mmol), 4-nitrobenzaldehyde (22.8 g, 150 mmol), copper acetate (Cu (OAc) 2) (908 mg, 5 mmol) and Cs₂CO₃ (48.9 g, 150 mmol) were introduced to a round bottom flask (RBF) . Dimethylformamide (DMF) (333 mL) was introduced thereto, and the mixture was stirred for 4 hours at 100°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/heptane to secure Intermediate 10-3 (45 g).

### 4) Synthesis of Intermediate 10-4

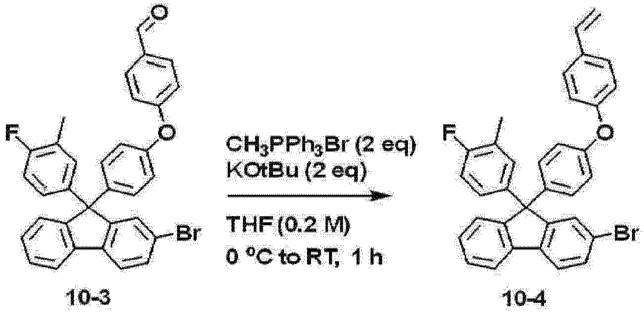

CH₃PPh₃Br (51.4 g, 144 mmol), potassium tert-butoxide (KOtBu) (16.2 g, 144 mmol) and tetrahydrofuran (THF) (216 mL) were introduced to a round bottom flask (RBF), and cooled to 0°C. A solution obtained by dissolving Intermediate 10-3 (39.6 g, 72 mmol) in tetrahydrofuran (THF) (144 mL) was introduced to the reaction mixture, and the result was stirred for 1 hour while raising the temperature to room temperature. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Intermediate 10-4 (35.9 g).

### 5) Synthesis of Compound 10

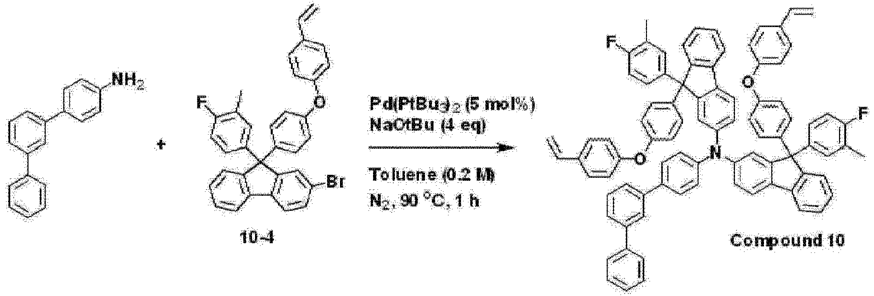

[1,1':3',1"-Terphenyl]-4-amine (687 mg, 2.8 mmol), Intermediate 10-4 (3.14 g, 5.74 mmol), Pd(PtBu₃)₂ (72 mg, 0.14 mmol) and sodium tert-butoxide (NaOtBu) (1.08 g, 11.2 mmol) were introduced to a round bottom flask (RBF). The flask was substituted with nitrogen, toluene (14 mL) was introduced thereto, and the mixture was stirred for 1 hour at 90°C. The result was extracted with ethyl acetate and water, and the organic layer was collected, dried using MgSO₄ and then filtered. The filtrate was dried using a vacuum rotary concentrator to remove the organic solvent. The result was column purified, and then crystallized under a condition of dichloromethane (DCM)/ethanol (EtOH) to secure Compound 10 (2.6 g). Synthesis of the compound was identified through LC-MS and NMR. MS: [M+H]⁺=1116
NMR measurement values of Compound 10: ¹H NMR (500 MHz, DMSO-d6) δ7.84-7.81 (m, 5H), 7.71 (d, 2H), 7.61-7.35 (m, 14H), 7.28 (t, 2H), 7.12-7.00 (m, 18H), 6.92 (d, 4H), 6.86 (t, 4H), 6.64 (m, 2H), 5.67 (dd, 2H), 5.17 (dd, 2H), 2.31 (s, 6H)

### <Device Example>

### Example 1

A glass substrate on which ITO (indium tin oxide) was coated as a thin film to a thickness of 1,500 Å was placed in detergent-dissolved distilled water and ultrasonic cleaned. Herein, a product of Fischer Co. was used as the detergent, and as the distilled water, distilled water filtered twice with a filter manufactured by Millipore Co. was used. After the ITO was cleaned for 30 minutes, ultrasonic cleaning was repeated twice using distilled water for 10 minutes. After the cleaning with distilled water was finished, the substrate was ultrasonic cleaned with solvents of isopropyl and acetone, dried, then washed for 5 minutes, and then transported to a glove box.

On a first electrode that is the transparent ITO electrode, a 2 wt% cyclohexanone ink including Compound 1 prepared in Preparation Example 1 and the following Compound G in a weight ratio of 8:2 was spin coated on the ITO surface, and heat treated for 30 minutes at 220°C to form a hole injection layer having a thickness of 400 Å.

On the hole injection layer, a 2 wt% toluene ink of the following Compound A was spin coated and heat treated for 10 minutes at 120°C to form a hole transfer layer having a thickness of 200 Å. The following Compound B and Compound C were vacuum deposited on the hole transfer layer in a weight ratio of 92:8 to form a light emitting layer having a thickness of 200 Å. The following Compound D was vacuum deposited on the light emitting layer to form an electron injection and transfer layer having a thickness of 350 Å. On the electron injection and transfer layer, LiF and aluminum were consecutively deposited to thicknesses of 10 Å and 1000 Å, respectively, to form a second electrode.

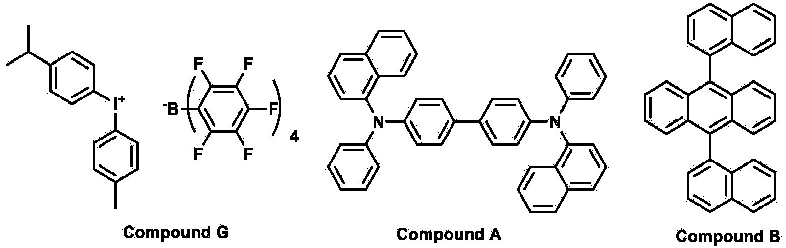

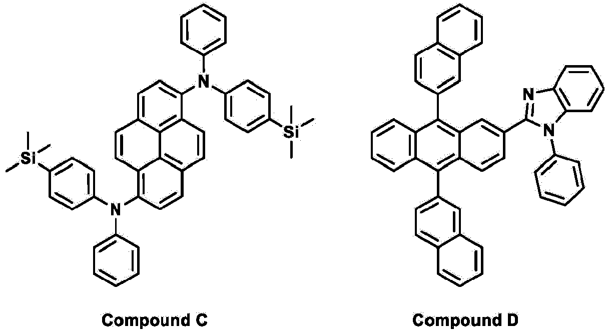

In the above-mentioned process, the deposition rates of the organic materials were maintained at 0.4 Å/sec to 0.7 Å/sec, the deposition rates of the lithium fluoride and the aluminum of the cathode were maintained at 0.3 Å/sec and 2 Å/sec, respectively, and the degree of vacuum during the deposition was maintained at 5×10⁻⁸ torr to 2×10⁻⁷ torr.

### Examples 2 to 10

Organic light emitting devices were manufactured in the same manner as in Example 1 except that the following Compounds 2 to 10 were used as described in the following Table 1 instead of Compound 1 when preparing the hole injection layer.

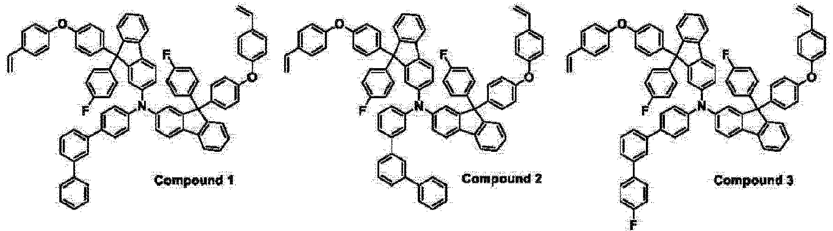

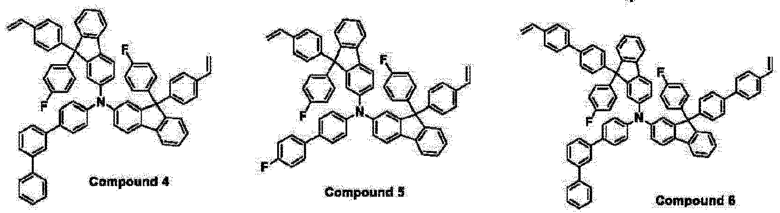

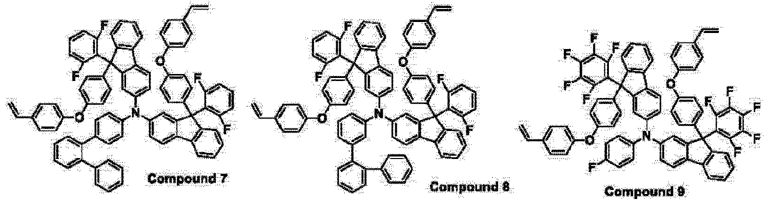

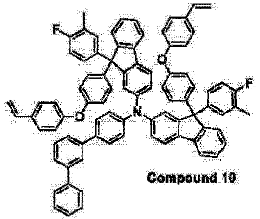

### Comparative Examples 1 to 4

Organic light emitting devices were manufactured in the same manner as in Example 1 except that any one compound of the following Compounds CE1 to CE4 was used as described in the following Table 1 instead of Compound 1 when preparing the hole injection layer.

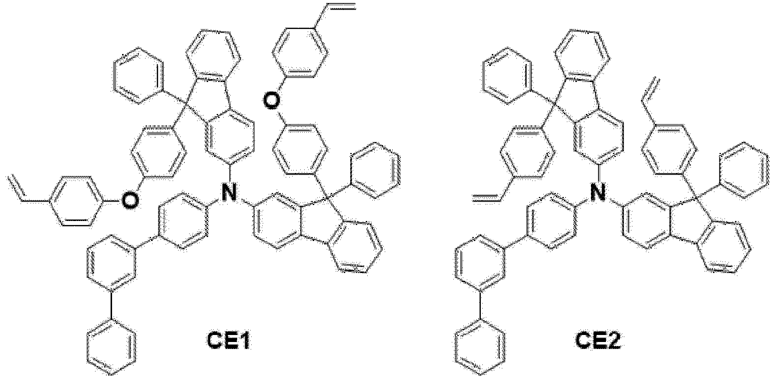

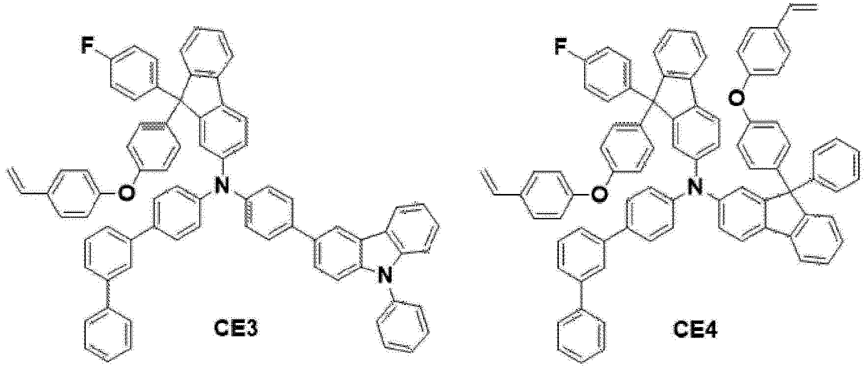

For each of the organic light emitting devices manufactured in the examples and the comparative examples, driving voltage, external quantum efficiency, luminance and lifetime were measured at current density of 10 mA/cm², and the results are shown in the following Table 1. The external quantum efficiency was obtained by (the number of emitted photons)/(the number of injected charge carriers). T95 means time (hr) taken for luminance to decrease to 95% from initial luminance (500 nit).

**[Table 1]**

| | HIL Host | Driving Voltage (V) | External Quantum Efficiency (%) | Luminan ce (cd/m²) | T95 (hr) @500 nit |
|---|---|---|---|---|---|
| Example 1 | Compound 1 | 4.61 | 5.9 | 591 | 182 |
| Example 2 | Compound 2 | 4.66 | 5.6 | 576 | 186 |
| Example 3 | Compound 3 | 4.71 | 5.8 | 589 | 173 |
| Example 4 | Compound 4 | 4.73 | 5.2 | 523 | 138 |
| Example 5 | Compound 5 | 4.78 | 5.7 | 580 | 151 |
| Example 6 | Compound 6 | 4.65 | 5.7 | 563 | 162 |
| Example 7 | Compound 7 | 4.83 | 5.5 | 548 | 133 |
| Example 8 | Compound 8 | 4.72 | 5.6 | 553 | 146 |
| Example 9 | Compound 9 | 4.58 | 5.3 | 542 | 169 |
| Example 10 | Compound 10 | 4.53 | 5.2 | 528 | 172 |
| Comparative Example 1 | CE1 | 5.40 | 4.5 | 436 | 68 |
| Comparative Example 2 | CE2 | 5.53 | 4.7 | 455 | 62 |
| Comparative Example 3 | CE3 | 6.11 | 5.1 | 464 | 47 |
| Comparative Example 4 | CE4 | 5.05 | 5.2 | 496 | 107 |

As shown in Table 1, it was identified that the organic light emitting devices of the examples had a significantly reduced driving voltage, and increased external quantum efficiency, luminance and lifetime compared to the organic light emitting devices of the comparative examples.

Specifically, Compounds 1 to 10 of Examples 1 to 10 improved properties of the film and/or the interface between the hole injection layer and the hole transfer layer in the organic light emitting device by including one nitrogen and two or more fluoro groups in the molecule, improved device properties by increasing stability of the fluoro group-substituted phenyl group, and had effects of improving hole mobility and hole/electron balance due to changes in the HOMO level. Accordingly, it was identified that the organic light emitting devices of Examples 1 to 10 using Compounds 1 to 10 as a host of the hole injection layer were effective in reducing a driving voltage, increasing external quantum efficiency, increasing luminance or increasing a lifetime.

On the other hand, Compounds CE1 to CE4 of Comparative Examples 1 to 4 include two or more nitrogen atoms, or only one or less fluoro group in the molecule, and it was identified that the organic light emitting devices including Compounds CE1 to CE4 did not have excellent effects that the organic light emitting devices including the compounds according to embodiments of the present disclosure had.

Specifically, Compounds CE1 and CE2 of Comparative Examples 1 and 2 do not include a fluoro group in the fluorenyl group bonding to the amine group. Accordingly, the organic light emitting devices including Compounds CE1 and CE2 had inferior effects of increased driving voltage, reduced external quantum efficiency, reduced luminance or reduced lifetime compared to the organic light emitting devices of Examples 1 to 10. Particularly, the organic light emitting devices including Compounds CE1 and CE2 had disadvantages of significantly reducing external quantum efficiency and luminance.

In addition, Compound CE3 of Comparative Example 3 includes only one fluoro group, and unlike other compounds, has two or more nitrogen atoms in the molecule by further including a carbazole group in the molecule. Accordingly, the organic light emitting device including Compound CE3 had inferior effects of increased driving voltage, reduced external quantum efficiency, reduced luminance or reduced lifetime compared to the organic light emitting devices of Examples 1 to 10. Particularly, the organic light emitting device including Compound CE3 had disadvantages of significantly increasing a driving voltage and significantly reducing a lifetime.

Furthermore, it was identified that Compound CE4 of Comparative Example 4 had inferior properties compared to Examples 1 to 10 of the present application by including only one fluorenyl group including a fluoro group.

## Claims

1. A compound represented by the following Chemical Formula 1: 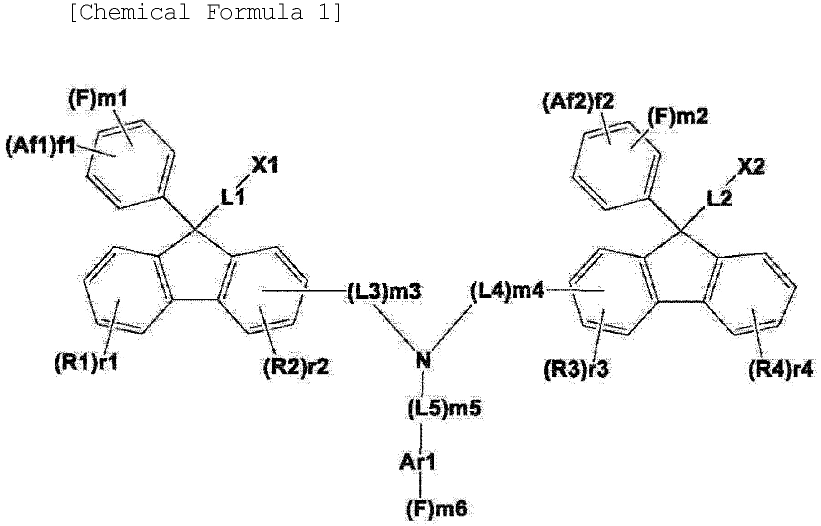 wherein, in Chemical Formula 1,
L1 and L2 are the same as or different from each other, and each independently a direct bond; a substituted or unsubstituted alkylene group; or a substituted or unsubstituted arylene group;
L3 to L5 are the same as or different from each other, and each independently a direct bond; or a substituted or unsubstituted arylene group;
R1 to R4 are the same as or different from each other, and each independently hydrogen; deuterium; a substituted or unsubstituted alkyl group; a substituted or unsubstituted alkoxy group; a substituted or unsubstituted aryl group; or a substituted or unsubstituted heteroaryl group;
Af1 and Af2 are the same as or different from each other, and each independently a substituted or unsubstituted alkyl group; or a substituted or unsubstituted aryl group;
Ar1 is a substituted or unsubstituted aryl group;
X1 and X2 are the same as or different from each other and each independently -(R101)s; or -Y-A, and at least one of X1 and X2 is -Y-A;
R101 is hydrogen; deuterium; a halogen group; a substituted or unsubstituted alkyl group; a substituted or unsubstituted alkoxy group; a substituted or unsubstituted aryl group; or a substituted or unsubstituted aryloxy group;
s is an integer of 0 to 5, and when s is 2 or greater, the two or more R101s are the same as or different from each other;
Y is a direct bond, O or S;
A is a curing group;
r1 and r4 are each an integer of 0 to 4;
r2 and r3 are each an integer of 0 to 3;
m1+m2 is an integer of 2 to 10;
m3 to m5 are each an integer of 0 to 2;
m6 is an integer of 0 to 5;
f1 and f2 are each an integer of 0 to 5, f1+m1 is 5 or less, and f2+m2 is 5 or less;
when r1 to r4 are each 2 or greater, substituents in the parentheses are the same as or different from each other; and
when m3 to m5 are each 2, substituents in the parentheses are the same as or different from each other.

2. The compound of Claim 1, wherein Chemical Formula 1 is represented by the following Chemical Formula 2: 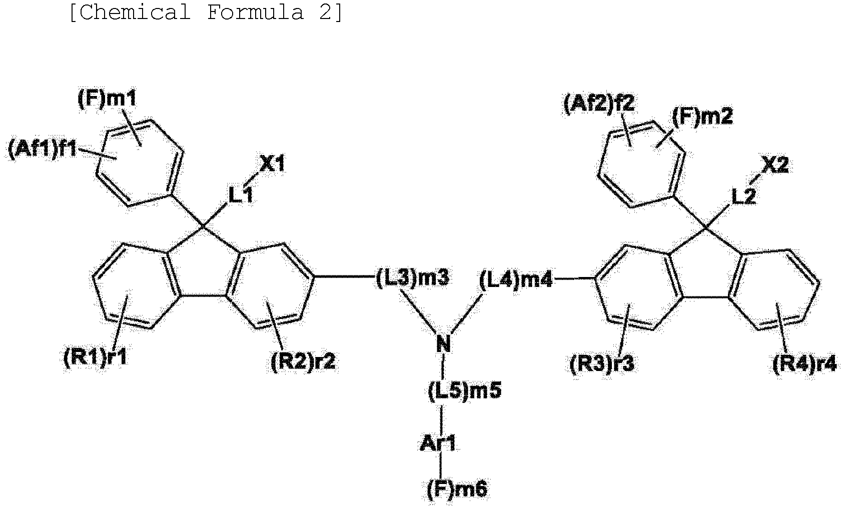 in Chemical Formula 2,
L1 to L5, R1 to R4, Af1, Af2, Ar1, X1, X2, r1 to r4, m1 to m6, f1 and f2 have the same definitions as in Chemical Formula 1.

3. The compound of Claim 1, wherein Chemical Formula 1 is represented by any one of the following Chemical Formulae 31 to 33: 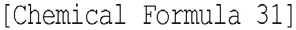 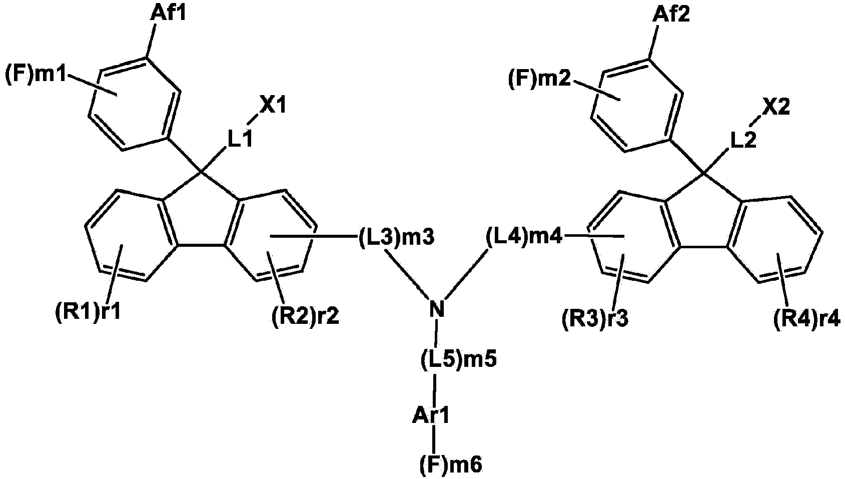 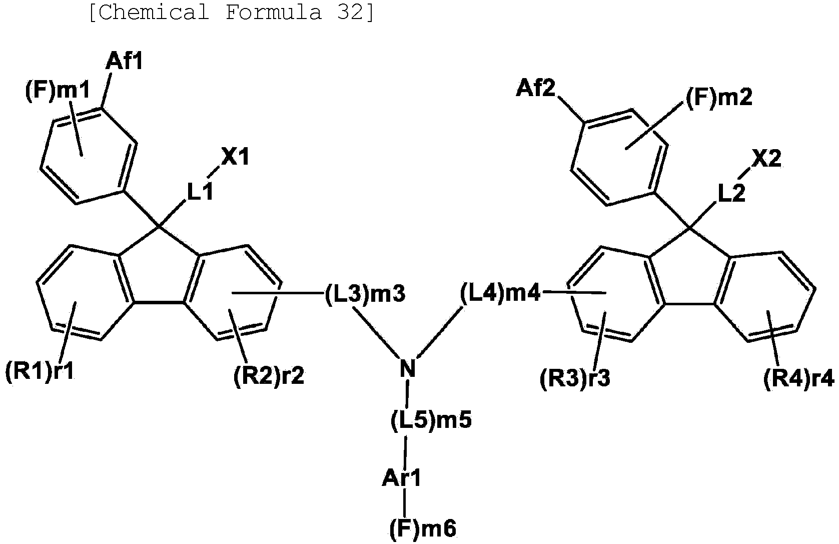 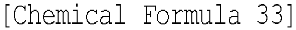 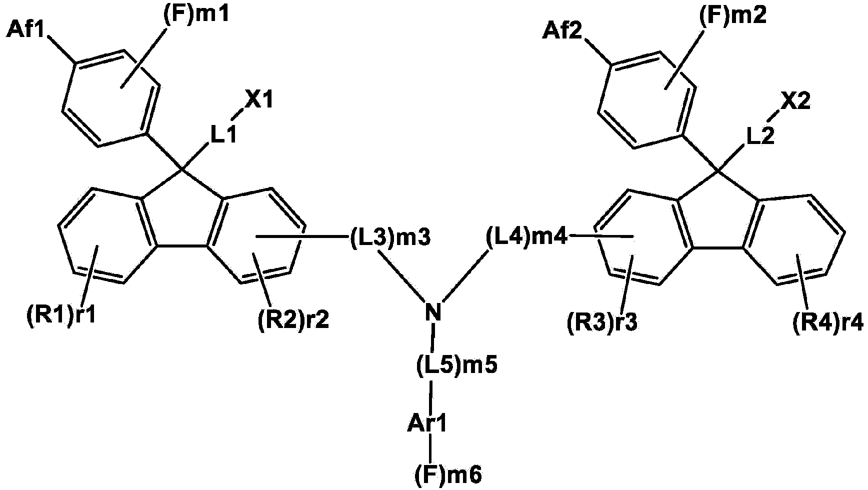 in Chemical Formulae 31 to 33,
L1 to L5, R1 to R4, Af1, Af2, Ar1, X1, X2, r1 to r4 and m1 to m6 have the same definitions as in Chemical Formula 1.

4. The compound of Claim 1, wherein A is any one selected from among the following structures: 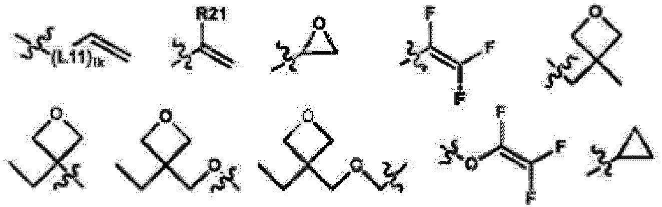 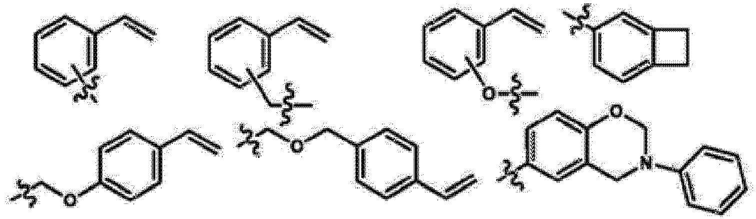 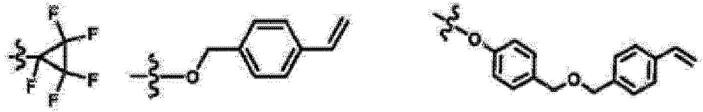 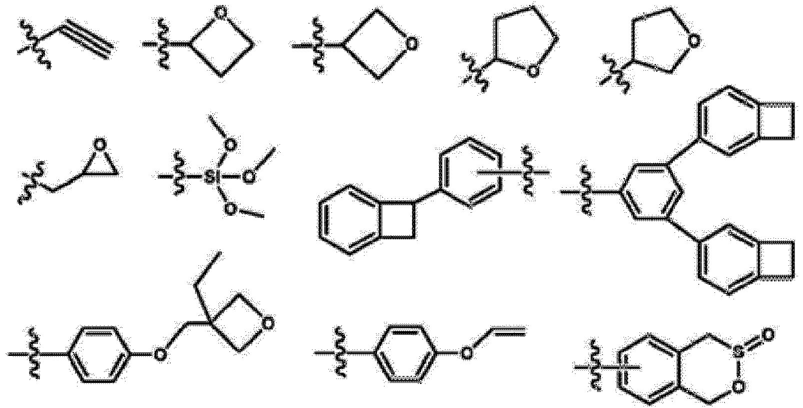 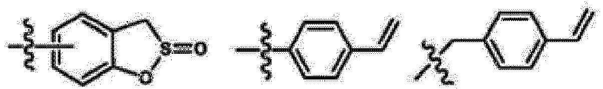 in the structures,
L11 is a direct bond; -O-; -S-; a substituted or unsubstituted alkylene group; a substituted or unsubstituted arylene group; or a substituted or unsubstituted heteroarylene group;
lk is 1 or 2;
when lk is 2, L11s are the same as or different from each other; and
R21 is a substituted or unsubstituted alkyl group.

5. The compound of Claim 1, wherein L1 and L2 are the same as or different from each other, and each independently a direct bond; a substituted or unsubstituted butylene group; a substituted or unsubstituted phenylene group; or a substituted or unsubstituted biphenylene group.

6. The compound of Claim 1, wherein L3 and L4 are a direct bond.

7. The compound of Claim 1, wherein L5 is a direct bond; a substituted or unsubstituted phenylene group; a substituted or unsubstituted biphenylene group; or a substituted or unsubstituted terphenylene group.

8. The compound of Claim 1, wherein Af1 and Af2 are the same as or different from each other, and each independently a substituted or unsubstituted methyl group; or a substituted or unsubstituted phenyl group.

9. The compound of Claim 1, wherein Ar1 is a substituted or unsubstituted phenyl group; a substituted or unsubstituted biphenyl group; or a substituted or unsubstituted terphenyl group.

10. The compound of Claim 1, wherein Chemical Formula 1 is any one selected from the group comprising the following compounds:

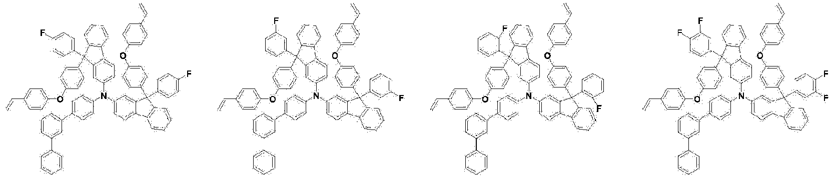

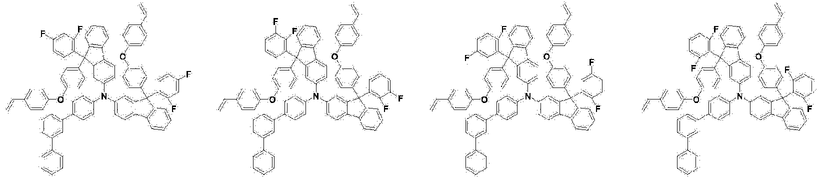

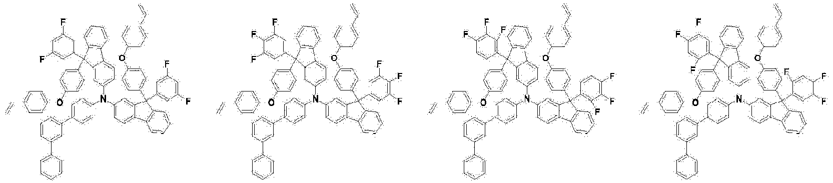

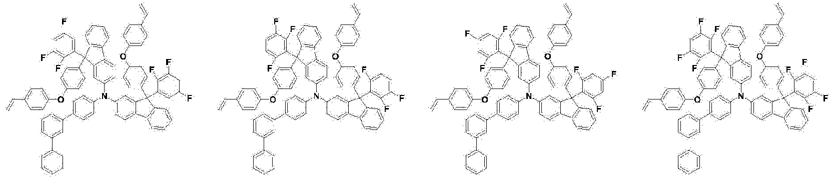

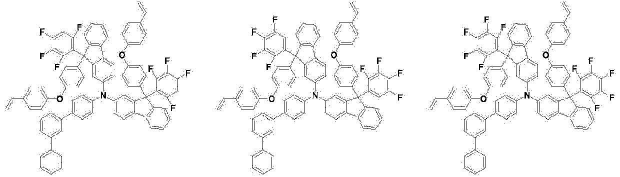

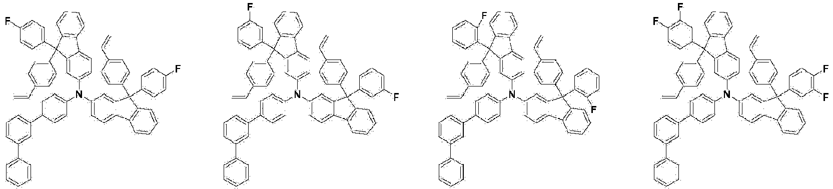

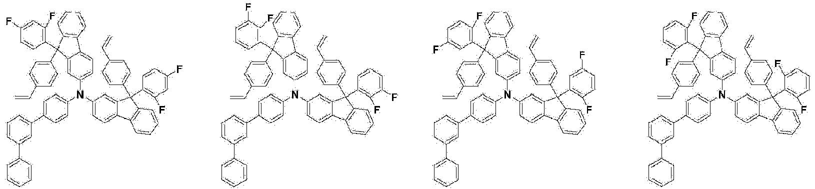

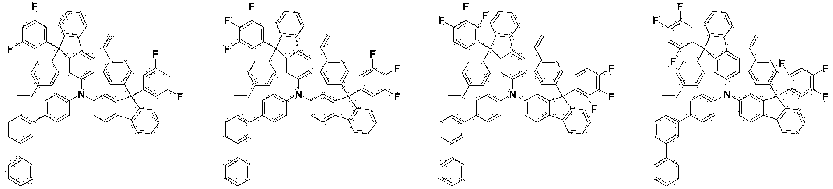

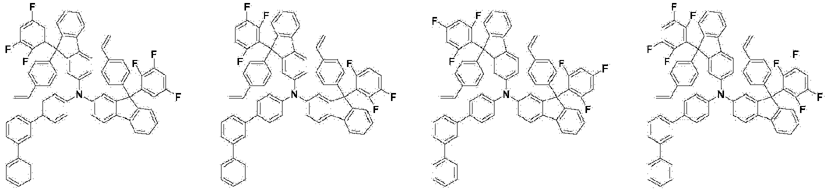

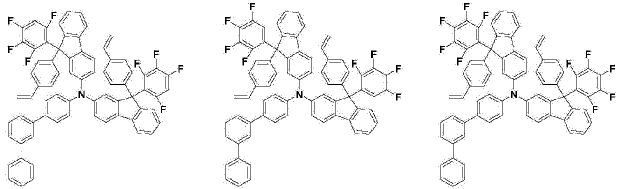

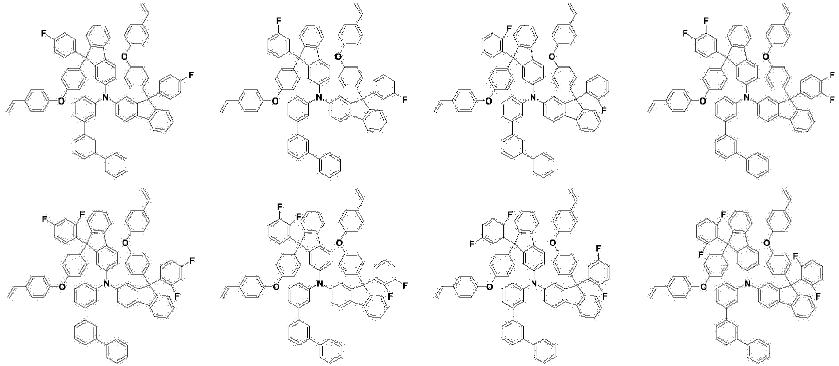

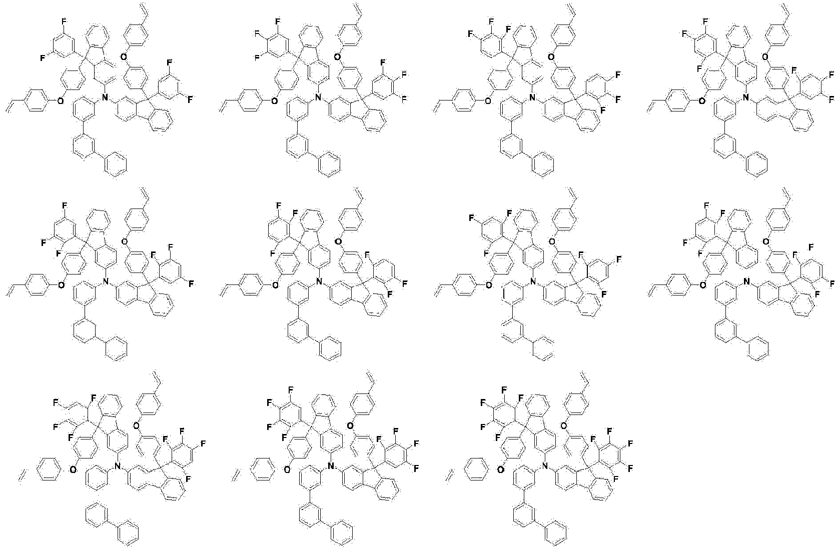

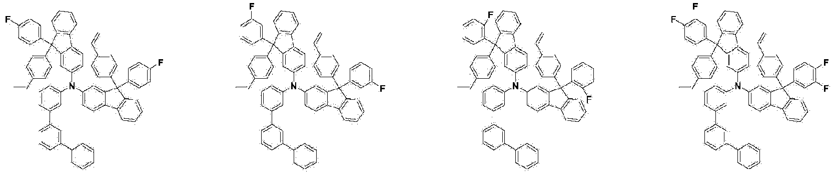

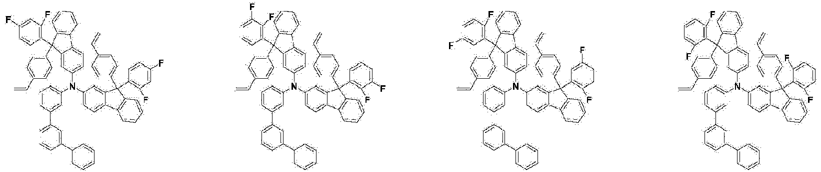

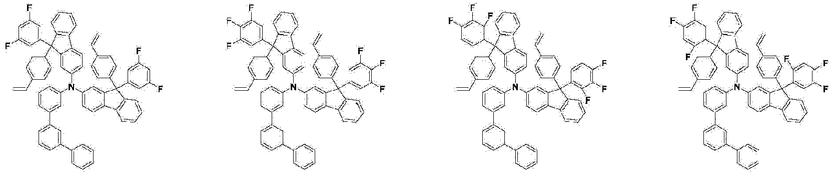

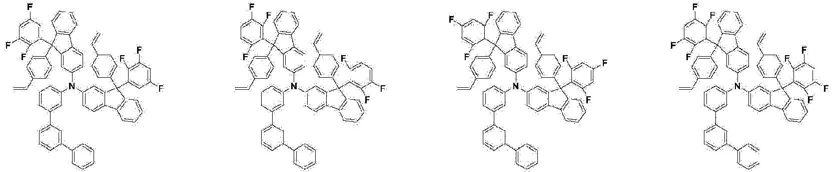

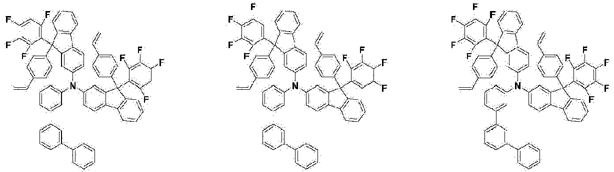

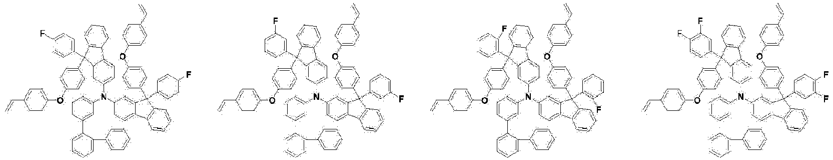

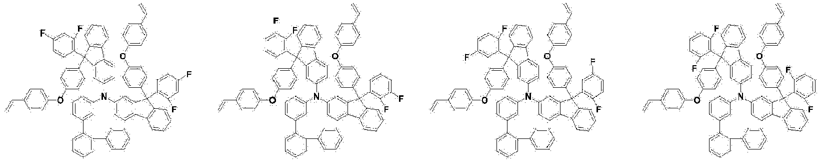

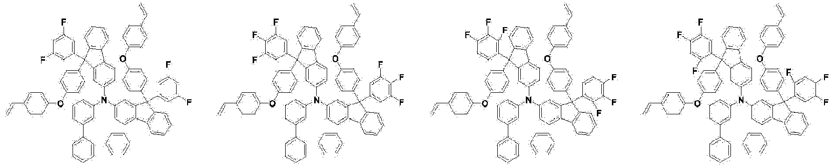

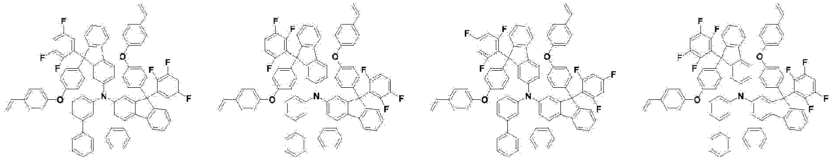

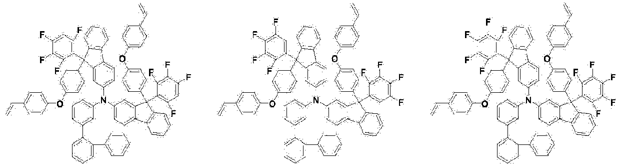

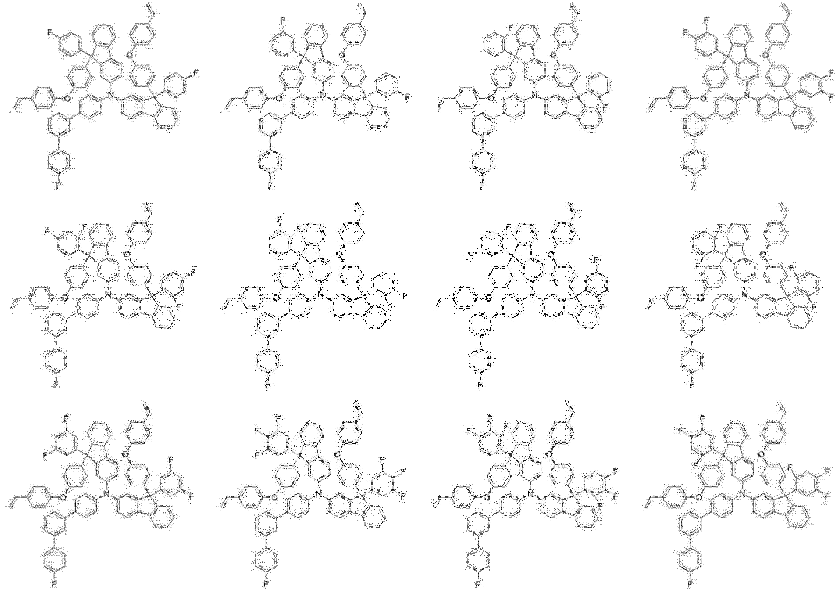

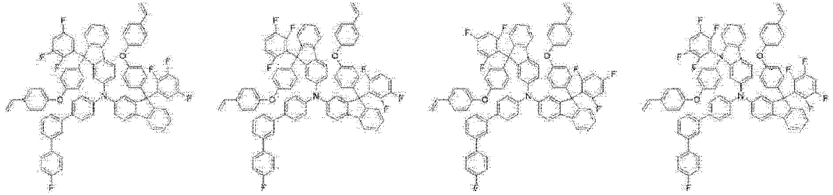

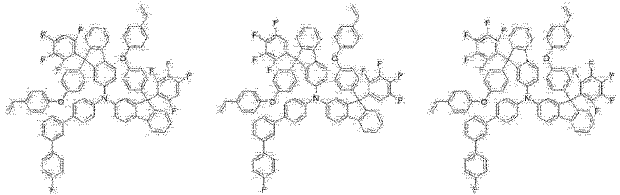

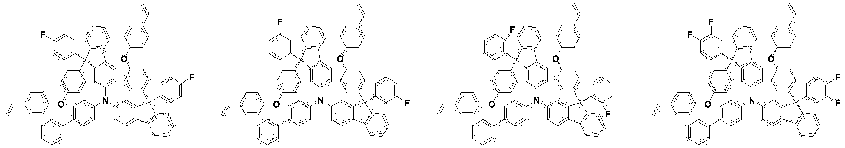

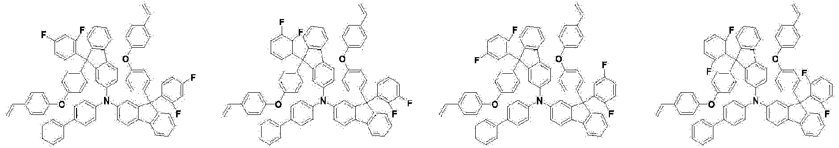

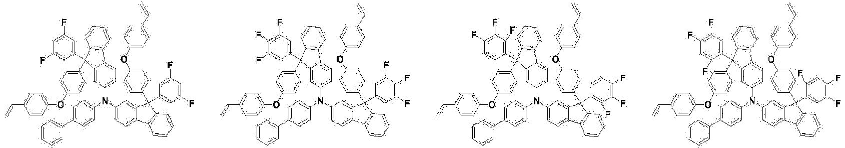

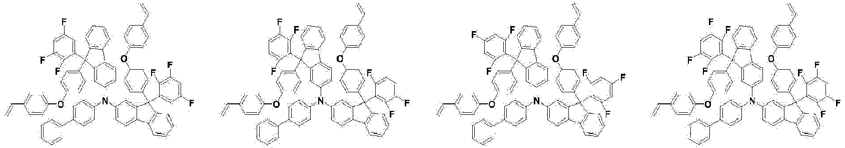

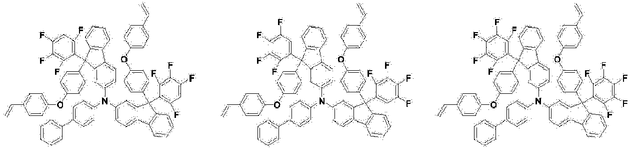

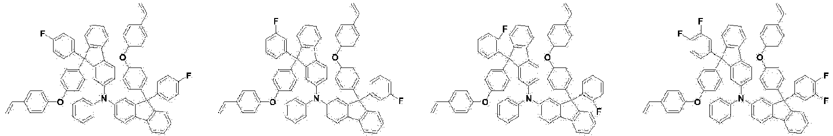

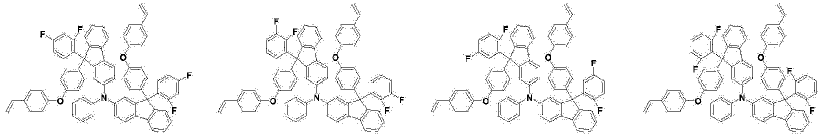

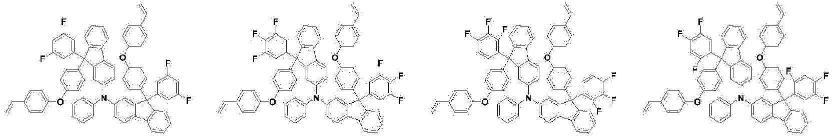

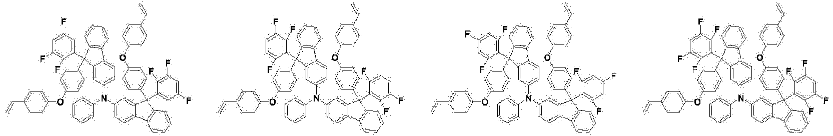

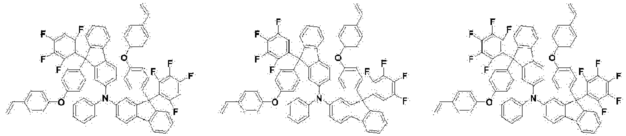

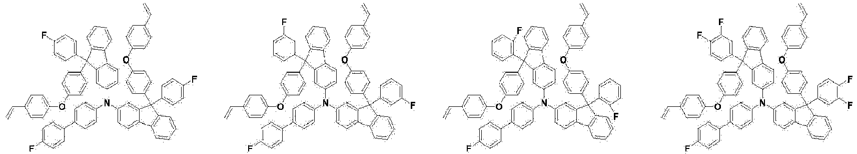

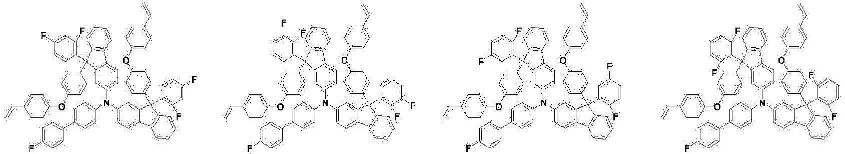

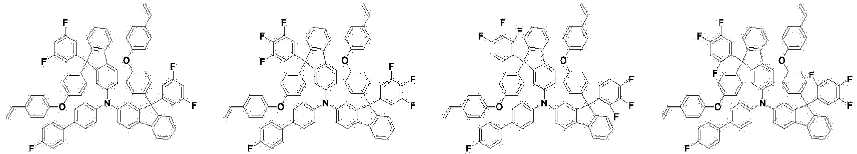

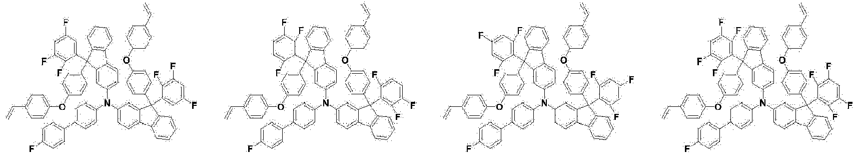

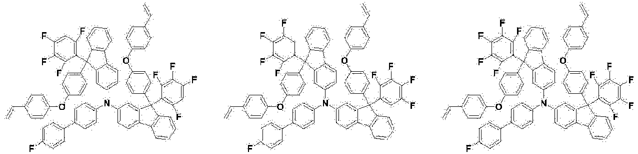

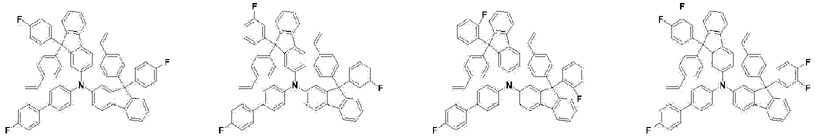

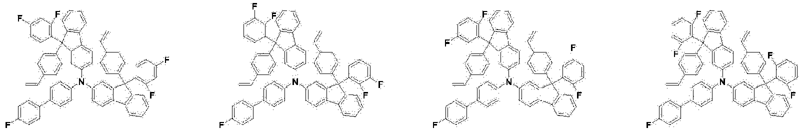

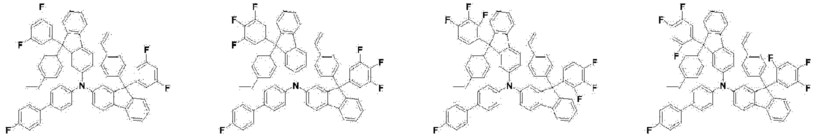

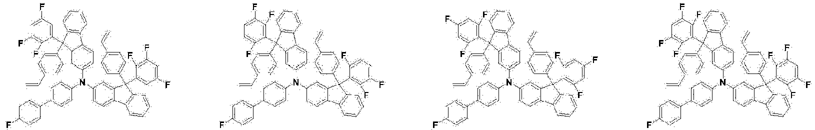

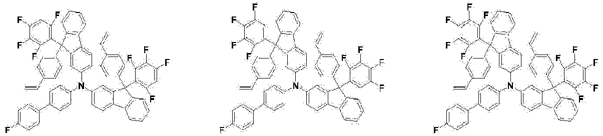

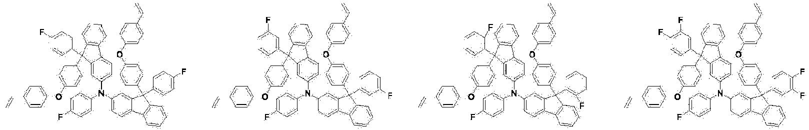

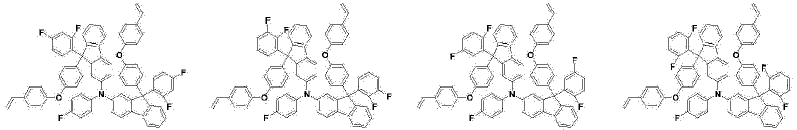

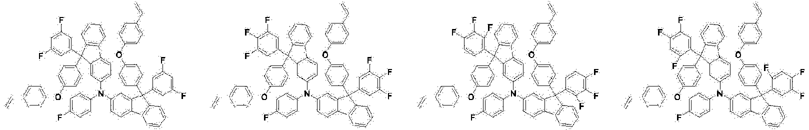

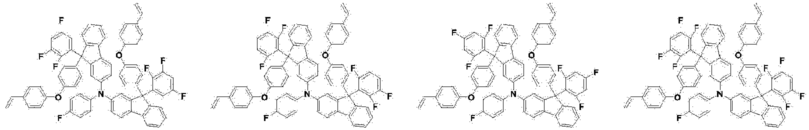

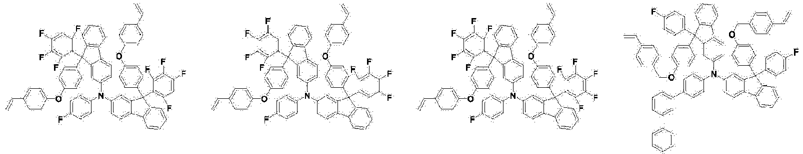

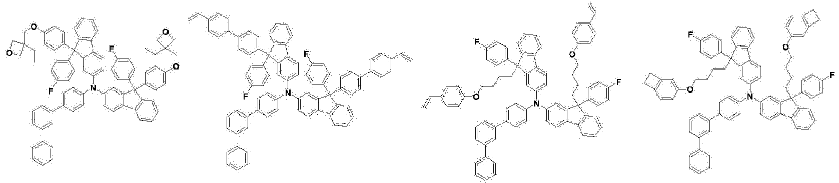

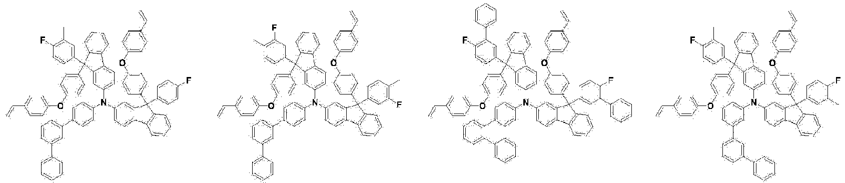

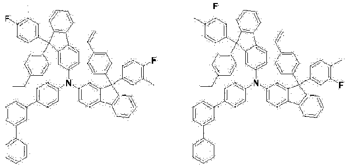

11. A coating composition comprising the compound of any one of Claims 1 to 10.

12. An organic light emitting device comprising:
a first electrode;
a second electrode; and
one or more organic material layers provided between the first electrode and the second electrode,
wherein one or more layers of the one or more organic material layers comprise the coating composition of Claim 11 or a cured material thereof.

13. The organic light emitting device of Claim 12, wherein the organic material layer comprising the coating composition or a cured material thereof is a hole injection layer.

14. The organic light emitting device of Claim 12, wherein the one or more organic material layers comprisie a hole injection layer, a hole transfer layer, a light emitting layer and an electron injection and transfer layer, and
the organic material layer comprising the coating composition or a cured material thereof is the hole injection layer.

15. A method for manufacturing an organic light emitting device, the method comprising:
preparing a first electrode;
forming one or more organic material layers on the first electrode; and
forming a second electrode on the one or more organic material layers,
wherein the forming of one or more organic material layers comprises forming one or more organic material layers using the coating composition of Claim 11.

16. The method for manufacturing an organic light emitting device of Claim 15, wherein the forming of organic material layers using the coating composition comprises,
coating the coating composition on the first electrode or the one or more organic material layers; and
heat treating or light treating the coated coating composition.
